# EUROPEAN PATENT APPLICATION

(11) **EP 4 772 632 A1**
(43) Date of publication of application: **08.07.2026**
(21) Application number: 23955860.4
(22) Date of filing: 31.10.2023
(51) Int. Cl.: C12N 15/113, C12N 15/10, C12N 9/00

(54) **GRNA AND BIOSYNTHESIS METHOD THEREFOR**

(30) Priority: 16.10.2023 CN 202311335106
(71) Applicant: Jilin Asymchem Laboratories Co., Ltd., Dunhua, Jilin 133700 (CN); Tianjin Asymchem Pharmaceuticals Co., Ltd., Tianjin 300462 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); PANG, Huining, Tianjin 300457 (CN); LIU, Fang, Tianjin 300457 (CN); MA, Cuiping, Tianjin 300457 (CN); WANG, Zhaoshuai, Tianjin 300457 (CN); JIA, Xu, Tianjin 300457 (CN); CUI, Lixin, Tianjin 300457 (CN); ZHU, Wenxuan, Tianjin 300457 (CN); ZHAO, Xiaolan, Tianjin 300457 (CN)
(74) Representative: Dehns Germany Partnerschaft mbB
(86) International application number: PCT/CN2023/128830
(87) International publication number: WO 2025/081542

(57) **Abstract**

Provided is gRNA and a method for biosynthesizing a gRNA thereof, which are suitable for the field of gRNA synthesis. The method includes: ligating 3' end and 5' end of different substrates to form a phosphodiester bond with an RNA ligase, so as to form the gRNA, where the gRNA includes natural gRNA or non-natural gRNA, and the number of the substrates is ≥2; the ligation site of the substrate is located at any one or more positions of a stem-loop structure complementary region, a stem-loop structure loop region, or a linker region between stem-loop structures of the gRNA, and wherein complementary base pairing occurs inside or between the substrates to form a secondary structure of the gRNA; and the RNA ligase includes any one or more enzymes of RNA ligase family 1, 2 or 3. The present disclosure can solves the problem in the related art of low purity of synthesized gRNA.

## Description

This application is based upon and claims priority to Chinese Patent Application No. 202311335106.2 filed on October 16, 2023, the disclosure of which is hereby incorporated by reference again in its entirety.

### Technical Field

The present disclosure relates to the field of gRNA synthesis, and specifically, to gRNA and a method for biosynthesizing a gRNA thereof.

### Background

Guide RNA (gRNA) is a fragment of non-coding RNA. In the 1980s, it was discovered in the mitochondria of kinetoplastid trypanosome in the order kinetoplastida of the class zoomastigophorea, and functions as a guide during RNA editing. The gRNA has garnered widespread attention stemming from the discovery and application of the CRISPR-Cas9 gene editing technology. The technology uses single guide RNA (sgRNA) to accurately locate a position to be edited on DNA, which is the Protospacer Adjacent Motif (PAM) site, causing Cas9 (DNA endonuclease) to identify the corresponding position for cleavage, thereby achieving the purpose of gene editing (deletion, insertion, or modification). The gRNA is single-stranded RNA that simultaneously contains crRNA and tracrRNA and has a length of approximately 67-142 nt. The crRNA is a variable sequence with a length of approximately 15-25 nt, and specifically recognizes a target gene for editing by means of complementary base pairing. The tracrRNA is a fixed sequence responsible for recruiting Cas9. Depending on the type of the Cas9, the sequence of the tracrRNA varies.

The most commonly used Cas9 protein currently is SpCas9 derived from *Streptococcus pyogenes.* Other available Cas9 proteins include SaCas9 derived from *Staphylococcus aureus,* FnCas9 derived from *Francisella novicida,* CjCas9 derived from *Campylobacter jejuni,* CdCas9 derived from *Corynebacterium diphtheriae,* St1Cas9 derived from *Streptococcus thermophilus,* and NmeCas9 derived from *Neisseria meningitidis.* The commonly used gRNA length for the SpCas9, SaCas9, FnCas9, and CjCas9 is approximately 100 nt. The commonly used gRNA length for the St1Cas9 and CdCas9 is approximately 115 nt. The maximum gRNA length available for the NmeCas9 is approximately 140 nt. Although the length of gRNA required by various Cas9 proteins is different, all gRNA shares a similar structure.

The gRNAs with a chain length of approximately 100 nt may be synthesized through in-vitro transcription and solid-phase synthesis methods in the prior art. However, existing solid-phase synthesis methods are mainly implemented through primer synthesis, with a production scale limited at a nmol-µmol level, making it difficult to scale up to a production scale. Furthermore, limited by the characteristic of solid-phase synthesis, an increase in chain length leads to decreased purity and yield, accompanied by problems such as reduced coupling efficiency, diminished carrier physical stability, increased branching impurities, etc. The gRNAs prepared by means of in-vitro transcription show inaccuracies in terminal sequences, affecting product quality.

### Summary

The present disclosure is mainly intended to provide gRNA and a method for biosynthesizing a gRNA thereof, so as to solve the problem in the prior art of low purity of synthesized gRNA.

In order to implement the above objective, according to a first aspect of the present disclosure, a method for biosynthesizing a gRNA is provided. The method includes: 3' end and 5' end of different substrates are ligated to form a phosphodiester bond with an RNA ligase, so as to form the gRNA, where the above gRNA includes a natural gRNA or a non-natural gRNA, and the number of the substrates is ≥2; a ligation site of substrates is located at any one or more positions of a stem-loop structure complementary region, a stem-loop structure loop region, or a linker region between stem-loop structures of the gRNA, complementary base pairing can be performed inside or between the substrates to form a secondary structure of the gRNA; and the RNA ligase includes any one or more enzymes of RNA ligase family 1, 2, or 3.

Further, the RNA ligase includes one or more of the RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24; or an enzyme having more than 70%, preferably, more than 70%, more than 75%, more than 80%, more than 90%, more than 95%, more than 99%, more than 99.5%, or more than 99.9% identity with the RNA ligase shown in any one of SEQ ID NO: 1-SEQ ID NO: 24.

Further, the number of the substrates is two or more.

Further, the gRNA has a length of 67-146 nt.

Further, the gRNA has a length of 91-104 or 130-146 nt.

Further, the sequence of the gRNA includes a nucleotide sequence shown in any one of SEQ ID NO: 25, SEQ ID NO: 26, SEQ ID NO: 27, SEQ ID NO: 28, SEQ ID NO: 41, SEQ ID NO: 42 or SEQ ID NO: 43.

Further, the substrates include: SEQ ID NO: 29 or a nucleotide sequence consisting of 15-25 N nucleotides; and nucleotide sequences shown in SEQ ID NO: 30 and SEQ ID NO: 31; or a nucleotide sequence as shown in SEQ ID NO: 32 or a sequence comprising the nucleotide sequence consisting of 15-25 N nucleotides and a nucleotide sequence as shown in SEQ ID NO: 36, and a nucleotide sequence as shown in SEQ ID NO: 33; wherein the nucleotide sequence consisting of 15-25 N nucleotides is in the 5' direction of the nucleotide sequence as shown in SEQ ID NO: 36; or a nucleotide sequence as shown in SEQ ID NO: 44 or a sequence comprising the nucleotide sequence consisting of 15-25 N nucleotides and a nucleotide sequence as shown in SEQ ID NO: 47, and nucleotide sequences as shown in SEQ ID NO: 45 and SEQ ID NO: 46; wherein the nucleotide sequence consisting of 15-25 N nucleotides is in the 5' direction of the nucleotide sequence as shown in SEQ ID NO: 36; or a nucleotide sequence as shown in SEQ ID NO: 48 or a sequence comprising the nucleotide sequence consisting of 15 to 25 N nucleotides and a nucleotide sequence as shown in SEQ ID NO: 50, and a nucleotide sequence as shown in SEQ ID NO: 49; wherein the nucleotide sequence consisting of 15-25 N nucleotides is in the 5' direction of the nucleotide sequence as shown in SEQ ID NO: 50.

Further, when the number of the substrates is 2, the method includes: the substrates and the RNA ligase are mixed, where the substrates include a first substrate and a second substrate, the RNA ligase links a phosphate group at the 5' end of the first substrate and a hydroxyl group at the 3' end of the second substrate to form the phosphodiester bond, thereby obtaining the gRNA.

Further, the 5' end of the second substrate is a 5' end protecting group, and the 3' end of the first substrate is a 3' end protecting group.

Further, when the number of the substrates is 3, the method includes: a first substrate, a second substrate, and the RNA ligase are mixed, where the 5' end of the first substrate is a phosphate group, the 3' end of the first substrate is a 3' end protecting group, and the 5' end and 3' end of the second substrate are hydroxyl groups; the phosphate group at the 5' end of the first substrate and the hydroxyl group at the 3' end of the second substrate are ligated to form the phosphodiester bond with the RNA ligase, so as to obtain an intermediate product of which the 5' end is a hydroxyl group; 5' phosphorylation is performed on the intermediate product to obtain a 5' phosphorylated intermediate product; and the 5' phosphorylated intermediate product, a third substrate and the RNA ligase are mixed, and the phosphate group at the 5' end of the 5' phosphorylated intermediate product and the hydroxyl group at the 3' end of the third substrate are ligated to form the phosphodiester bond with the RNA ligase, so as to obtain the gRNA, where the 5' end of the third substrate is a 5' end protecting group.

Further, by repeatedly performing ligation and 5' phosphorylation and sequentially ligating the substrates via the method, four or more substrates are subjected to multiple rounds of ligation and 5' phosphorylation. Two substrates are used in each ligation step, and the substrates are sequentially ligated to obtain the gRNA.

Further, a template nucleic acid strand is used to guide the ligating of the substrates; the template nucleic acid strand has at least 3 base specific bindings with each of different substrates, the bases of the substrates specifically binding to the template nucleic acid chain are adjacent and form a nick, forming a double-stranded nucleotide structure containing the nick, bases adjacent to two ends of the nick are a 5' end and a 3' end of different substrates, the 5' end is a 5' phosphate, and the 3' end is a hydroxyl group; the 5' phosphate and the hydroxyl group at upstream and downstream of the nick are ligated to form the phosphodiester bond with the RNA ligase, so as to obtain a double-stranded nucleotide structure; and the template nucleic acid strand of the double-stranded nucleotide structure is digested with a DNase to obtain the gRNA.

Further, a sequence of the template nucleic acid strand includes any one of the nucleotide sequences as shown in SEQ ID NO: 37, SEQ ID NO: 39, SEQ ID NO: 40, or SEQ ID NO: 51.

Further, after mixing the template nucleic acid strand with different substrates, the mixture is subjected to high temperature incubation followed by slow cooling for annealing, or to constant temperature incubation, thereby obtaining the double-stranded nucleic acid structure containing a nick. A temperature of the constant temperature incubation is 4-37 °C, and a time of the constant temperature incubation is ≥10 min; and performing high temperature incubation and then performing slow cooling and annealing includes: incubation is performed at 95 °C for 2 min, the temperature is reduced to 12 °C at a rate of 0.5-1.2 °C/min, incubation is performed at 12 °C for 10 min, and then the temperature is reduced to 4 °C.

Further, the 3' end of the substrate at the 3' end of the gRNA is a 3' end protecting group, and the 5' end of the substrate at the 5' end of the gRNA is a 5' end protecting group.

Further, the secondary structure of the gRNA is formed by the substrates themselves or by spontaneous complementary base pairing between the substrates.

Further, the 3' end protecting group includes, but is not limited to, modification groups, such as an N-acetylgalactosamine group, a phosphate group, -O-NH₂, an azide group, a cyanoethyl group, an allyl group or a 2-nitrobenzyl group, etc., that can prevent the formation of a phosphoester bond; and the 5' end protecting group includes, but is not limited to, modification groups, such as hydroxyl group, hydrogen or methyl group, etc., that can prevent the formation of the phosphoester bond.

Further, the substrate includes one or more non-natural nucleotides, and the non-natural nucleotide includes: a ribonucleotide having one or more of a ribose 2' position modification, a ribose backbone modification, a base modification or a phosphate backbone modification; the ribose position 2' modification includes a 2'-methoxy modification, a 2'-fluoro modification, a 2'-hydrogen modification, a 2'-methoxyethyl modification, a 2'-FANA modification, a locked nucleic acid modification, or a hexitol nucleic acid modification; the ribose backbone modification includes replacing a ribose in the nucleotide with ribuloNA, TNA, tPhoNA, or dXNA; the base modification includes a deazaadenine C7 modification, a deazaguanosine C7 modification, a cytosine C5 modification or a uridine C5 modification; and the phosphate backbone modification includes a PS modification.

In order to implement the above objective, according to a second aspect of the present disclosure, gRNA is provided. The gRNA includes the gRNA obtained through preparation using the above method.

With the technical solutions of the present disclosure, with the above method, two or more than two substrates are ligated by the RNA ligases of the RnI1, RnI2, or Rnl3 family, a nick position for ligating may be located on the stem-loop structure of the gRNA, or may also be located to the ligating region between the stem-loop structures, such that the plurality of substrates are ligated into a complete RNA strand, and complementary base pairing can be automatically performed between the substrates, thereby obtaining the gRNA having a secondary structure through preparation. The above method can perform the reaction with a high substrate concentration, achieves a high yield, and thus can meet requirements for industrial large-scale production.

### Brief Description of the Drawings

The drawings, which form a part of the present application, are used to provide a further understanding of the present disclosure. The exemplary embodiments of the present disclosure and the description thereof are used to explain the present disclosure, but do not constitute improper limitations to the present disclosure. In the drawings:
Fig. 1 is a schematic diagram of reactions according to Embodiments 1-3 of the present disclosure, where A is a schematic diagram of the splicing of three fragments using RNA ligases, B is a schematic diagram of the splicing of two fragments using the RNA ligases, and C is a schematic diagram of fragment splicing using the RNA ligases under the guidance of a template DNA strand.
Fig. 2 is a schematic diagram of a secondary structure of SEQ ID NO: 26 according to Embodiment 1 of the present disclosure.
Fig. 3 is a schematic diagram of a secondary structure of SEQ ID NO: 28 according to Embodiment 1 of the present disclosure.
Fig. 4 is a diagram of gel electrophoresis results of S1 according to Embodiment 1 of the present disclosure.
Fig. 5 is a diagram of UPLC results of S1 according to Embodiment 1 of the present disclosure.
Fig. 6 is a diagram of UPLC results of S2 according to Embodiment 1 of the present disclosure.
Fig. 7 is a diagram of gel electrophoresis results of S3 according to Embodiment 1 of the present disclosure.
Fig. 8 is a diagram of UPLC results of S3 according to Embodiment 1 of the present disclosure.
Fig. 9 is a diagram of gel electrophoresis results according to Embodiment 2 of the present disclosure.
Fig. 10 is a diagram of UPLC results according to Embodiment 2 of the present disclosure.
Fig. 11 is a diagram of gel electrophoresis results according to Embodiment 3 of the present disclosure.
Fig. 12 is a diagram of UPLC results according to Embodiment 3 of the present disclosure.
Fig. 13 is a diagram of UPLC results of S1 according to Embodiment 4 of the present disclosure.
Fig. 14 is a diagram of UPLC results of S3 according to Embodiment 4 of the present disclosure.
Fig. 15 is a diagram of UPLC results according to Embodiment 5 of the present disclosure.
Fig. 16 is a diagram of UPLC results according to Embodiment 6 of the present disclosure.

### Detailed Description of the Embodiments

It is to be noted that the embodiments in the present application and the features in the embodiments may be combined with one another without conflict. The present disclosure will be described below in detail with reference to the embodiments.

As mentioned in the Background, methods for synthesizing gRNA in the prior art is low in purity, low in production efficiency, difficult in product purification, and high in cost, and thus is difficult to meet requirements for industrial large-scale production. The inventor in the present application attempts to develop a method for biosynthesizing a gRNA, thereby proposing a series of protective solutions of the present application.

In a first typical implementation of the present application, a method for biosynthesizing a gRNA is provided. The method includes: the 3' end and the 5' end of different substrates are ligated to form a phosphodiester bond with an RNA ligase, so as to form the gRNA, where the above gRNA includes a natural gRNA or a non-natural gRNA, and the number of the substrates is ≥2; a ligation site of substrates is located at any one or more positions of a stem-loop structure complementary region, a stem-loop structure loop region, or a linker region between stem-loop structures of the gRNA, complementary base pairing can be performed inside or between the substrates to form a secondary structure of the gRNA; and the RNA ligase includes any one or more enzymes of RNA ligase family 1, 2 or 3.

In a preferred embodiment, the RNA ligase includes one or more of the RNA ligases shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23, and SEQ ID NO: 24; or an enzyme having more than 70%, preferably, more than 70%, more than 75%, more than 80%, more than 90%, more than 95%, more than 99%, more than 99.5%, or more than 99.9% identity with the RNA ligase shown in any one of SEQ ID NO: 1-SEQ ID NO: 24.

The ligating between 3' hydroxyl group of the substrate and 5' phosphate can be catalyzed to form a phosphodiester bond with the above RNA ligase, thereby preparing a natural gRNA or a non-natural gRNA. In the present application, the natural gRNA is gRNA consisting of unmodified natural nucleotides A, C, G, and U; and the non-natural gRNA is gRNA containing a non-natural nucleotide. Natural nucleic acids can be ligated with the above RNA ligase, and non-natural nucleic acids can also be ligated. A nick of the above ligating may be in a stem-loop structure (including a stem-loop structure complementary region and a stem-loop structure loop region) of the gRNA and/or a ligating region between the stem-loop structures. The above RNA ligases all can achieve ligating activity. The above substrates have sequences that are capable of base complementary pairing with each other or with themselves. Complementary base pairing can automatically occur before, during, or after the ligating using the RNA ligases, so as to generate a spatial structure, thereby forming the gRNA having a secondary structure.

The RNA ligases include the followings.
ligase-1, SEQ ID NO: 1, *Bacteriophage* T4, RNA ligase 1 (RNA ligase family 1, Rnl1): 
MQELFNNLMELCKDSQRKFFYSDDVSASGRTYRIFSYNYASYSDWLLPDALECRGIMFEMDG
EKPVRIASRPMEKFFNLNENPFTMNIDLNDVDYILTKEDGSLVSTYLDGDEILFKSKGSIKSEQALMA
NGILMNINHHRLRDRLKELAEDGFTANFEFVAPTNRIVLAYQEMKIILLNVRENETGEYISYDDIYKDA
TLRPYLVERYEIDSPKWIEEAKNAENIEGYVAVMKDGSHFKIKSDWYVSLHSTKSSLDNPEKLFKTII
DGASDDLKAMYADDEYSYRKIEAFETTYLKYLDRALFLVLDCHNKHCGKDRKTYAMEAQGVAKGA
GMDHLFGIIMSLYQGYDSQEKVMCEIEQNFLKNYKKFIPEGY.
ligase-2, SEQ ID NO: 2, *Salmonella phage* STP4-a, RNA ligase 2 (RNA ligase family 2, RnI2): 
MFKKYSSLENHYNNKFISKIRFEGKDGGLWVAREKIHGTNFSIIVSKDSVSACKRSGPILPSES
FYGHEIILKNYDESIKTIQRCMNANELGSVSSYQIFGEFAGQGIQKEVDYGEKDFYVFDILVNTQNG
NVLYMDDMMMTSFCNEFGFKMAPFIGCGSFDELIQLPNNFTSVIKAYNEAAKDDLKEVNLCVFDPL
VTDDNVAEGYVLKPVYPDFFNNGTRIAIKSKNSRFTEKKKSDKPIKPKAVLTSNDSTVLANLCEYST
WNRVSNVISHIGEVKAKDFGKVMGLTIQDIFIEAKREGVDIVHADNPDLVKRELQTVVSNTIREKWL
EIVS.
ligase-3, SEQ ID NO: 3, *Bacteriophage* T4, RNA ligase 2: 
MFKKYSSLENHYNSKFIEKLYSLGLTGGEWVAREKIHGTNFSLIIERDKVTCAKRTGPILPAEDF
FGYEIILKNYADSIKAVQDIMETSAVVSYQVFGEFAGPGIQKNVDYCDKDFYVFDIIVTTESGDVTYV
DDYMMESFCNTFKFKMAPLLGRGKFEELIKLPNDLDSVVQDYNFTVDHAGLVDANKCVWNAEAK
GEVFTAEGYVLKPCYPSWLRNGNRVAIKCKNSKFSEKKKSDKPIKAKVELSEADNKLVGILACYVTL
NRVNNVISKIGEIGPKDFGKVMGLTVQDILEETSREGITLTQADNPSLIKKELVKMVQDVLRPAWIEL
VS.
ligase-4, SEQ ID NO: 4, *Vibrio phage* KVP40, RNA ligase 2: 
MSFVKYTSLENSYRQAFVDKCDMLGVREWVALEKIHGANFSFIVEFKPNEAQDGAEFTVTPA
KRTSTIGANVMGDYDFYGCTSVVEAHTAKMEAISNWLWARGIINVGETIIVYGELAGKGVQKEVNY
GDKDFWAYDILLPETGKFLDWDVVLEACEFAKVKTTHEIARGTLDELLRIDPLFRSFHTPADVDGDN
VAEGFVVKQLRNEKRLHNGSRAILKVKNDKFKEKKNKAGKTPRAAVVLTEEQEKLHAAFSCYLTEN
RLRNVLSKLETVTQKQFGMISGLFIKDAKDEFERDELNETAIARDDWDVIKRSLTNIANEILRKNWLD
ILDGNF.
ligase-5, SEQ ID NO: 5, *Pyrococcus abyssi,* RNA ligase 3 (RNA ligase family 3, Rnl3): 
MVSSVYKEILVKLGLTEDRIETLEMKGGIIEDEFDGIRYVRFKDSAGKLRRGTVVIDEEYVIPGF
PHIKRIINLRSGIRRIFKRGEFYVEEKVDGYNVRVVMYKGKMLGITRGGFICPFTTERIPDFVPQEFF
KDNPNLILVGEMAGPESPYLVEGPPYVKEDIQFFLFDVQEIKTGRSLPVEERLKIAEEYGINHVEVF
 
GKYTKDDVDELYQLIERLSKEGREGIIMKSPDMKKIVKYVTPYANINDIKIGARVFYELPPGYFTSRIS
RLAFYLAEKRIKGEEFERVAKELGSALLQPFVESIFDVEQEEDIHELFKVRVKRIETAYKMVTHFEKL
GLKIEIVDIEEIKDGWRITFKRLYPDATNEIRELIGGKAFVD.
ligase-6, SEQ ID NO: 6, *Vibrio phage* nt-1, RNA ligase 2: 
MSFVKYTSLENSYRQAFVDKCDMLGVRDWVALEKIHGANFSFIVEFDGGYTVTPAKRTSIIGA
TATGDYDFYGCTSVVEAHKEKVELVANFLWLNEYINLYEPIIIYGELAGKGIQKEVNYGDKDFWAFDI
FLPQREEFVDWDTCVAAFTNAEIKYTKELARGTLDELLRIDPLFKSLHTPAEHEGDNVAEGFVVKQL
HSEKRLQSGSRAILKVKNEKFKEKKKKEGKTPTKLVLTPEQEKLHAEFSCYLTENRLKNVLSKLGTV
NQKQFGMISGLFVKDAKDEFERDELNEVAIDRDDWNAIRRSLTNIANEILRKNWLNILDGNF.
ligase-7, SEQ ID NO: 7, *Shigella phage* Sf22, RNA ligase 1: 
MQELFNNLMELCKDSQRKFFYSDDVSASGRTYRIFSYNYASYSDWLLPDALECRGIMFEMDG
EKPVRIASRPMEKFFNLNENPFTMNIDLNDVDYILTKEDGSLVSTYLDGDEILFKSKGSIKSEQALMA
NGILMNINHHQLRDRLKELAEDGFTANFEFVAPTNRIVLAYQEMKIILLNIRENETGEYISYDDIYKDA
ALRPYLVERFEVDSPKWIEEAKNAENIEGYVAVMKDGSHFKIKSDWYVSLHSTKSSLDNPEKLFKTI
IDGASDDLKAMYADDEYSYRKIEAFETTYLKYLDRALFLVLDCHNKHCGKDRKTYAMEAQGVAKGA
GMEHLFGIIMSLYQGYDSQEKVMCEIEQNFLKNYKKFIPEGY.
ligase-8, SEQ ID NO: 8, *Vibrio phage* VH7D, RNA ligase 1: 
MNVQELYKNLMSLADDAEGKFFFADHLSPLGEKFRVFSYHIASYSDWLLPGALEARGIMFQLD
DNDEMIRIVSRPMEKFFNLNENPFTMELDLTTTVQLMDKADGSLISTYLSGENFALKSKTSIFSEQA
VAANRYIKKPENRDLWEFCDDCTQAGLTVNMEWCAPNNRIVLEYPEAKLVILNIRDNETGDYVSFD
DIPQSALMRVKQWLVDEYDPATAHEPDFVEKLRDTKGIEGMILRLANGQSVKIKTQWYVDLHSQKD
SVNVPKKLVTTILNGNHDDLYALFADDKPTIERIREFDSHVTKTLTNSFNAVRQFYARNRHLARKDYA
IAGQKVLKPWEFGVAMIAYQKQTVEGVYESLVTAYLKRPELAIPEKYLNGV.
ligase-9, SEQ ID NO: 9, *Escherichia phage* dhaeg, RNA ligase 2: 
MFKKYSSLENHYNSKFIEKLRTNGLTGGEWVAREKIHGTNFSLIIERDAVTCAKRTGPILPAEDF
YGYEIVLKNYADSIKSIQDIMETSAAVSYQVFGEFAGTGIQKNVDYGDKDFYVFDIIVTTESGDVTYV
DDYMMESFCKTFKFKMAPLLGRGKFEDLIKLPNDLDSVLPDYNFTVDNVGLAEANAHVWNAEAK
GEVFTAEGYVLKPCYPLWLPNGNRVAIKCKNSKFSEKKKTDKPIKVAVVLSQDDLDLLQQFTDYVT
VNRINNVISKIGEVSPKDFGKVMGLTVQDILEEAAREELELTDAENPVEVKKQLIECVKDTLRAVWIE
LVSK.
ligase-10, SEQ ID NO: 10, *Klebsiella phage* KP27, RNA ligase 2: 
MFKKYSSLTNHYEGKFINGVIMNGLTGGVWVAREKIHGANFSFITDDGITVTPAKRTDVVKPAE
DFYGCSAVVAKYSPGIRKMWETLKKTGTYDDLVIQVYGEFAGRGVQKDVDYGEKDFYVFDIRVNG
EFLPDNLCSLISRSHGLKMAPLLGYGTFEEIKELPITFESVVNKANSGIGSDNTVYGEFVYPIMDVEE
 
GNIAEGFVMKPVSPAFMPNGERVAIKCKTTKFTEKKAKKATRFNAPVSLSEKDKNQLDEFVCYLTE
NRVKNVLSKLDLASITAKDFGRIMGLTVQDAIEEISRNHGPFLEQFEDPAMAKKLFVTEAQNMIRPV
WGKILNHEF.
ligase-11, SEQ ID NO: 11, *Vibrio phage V05,* RNA ligase 1: 
MTTQELYNHLMTLTDDAEGKFFFADHISPLGEKLRVFSYHIASYSDWLLPGALEARGIMFQLD
EQDKMVRIVSRPMEKFFNLNENPFTMDLDLTTTVQLMDKADGSLISTYLTGENFALKSKTSIFSEQA
VAANRYIKLPENRDLWEFCDDLTQAGCTVNMEWCAPNNRIVLEYPEAKLVILNIRDNETGDYVSFD
DIPLPALMRVKKWLVDEYDPETAHADDFVEKLRATKGIEGMILRLANGQSVKIKTQWYVDLHSQKD
SVNVPKKLVTTILNNNHDDLYALFADDKPTIDRIREFDSHVSKTVSASFHAVSQFYVKNRHMSRKDY
AIAGQKTLKPWEFGVAMIAYQNQTVEGVYEALVGAYLKRPELLIPEKYLNEA.
ligase-12, SEQ ID NO: 12, *Escherichia phage* AR1, RNA ligase 2: 
MFKKYSSLENHYNSKFIEKLYSLGLTGGEWVAREKIHGTNFSLIIERDKVTCAKRTGPILPAEDF
FGYEIILKNYADSIKAVQDIMETSAVVSYQVFGEFAGPGIQKNVDYGDKDFYVFDIIVTTESGDMTYV
DDYMMESFCNTFKFKMAPLLGRGKFEELIKLPNDLDSVVQDYNFTVDHAGLVDANKCVWNAEAK
GEVFTAEGYVLKPCYPSWLPNRNRVAIKCKNSKFSEKKKSDKPIKAKVELSEADNKLVGILACYVTL
NRVNNVISKIGEIGPKDFGKVMGLTVQDILEETSREGITLTQADNPSLIKKELVKMVQDVLRPAWIEL
VS.
ligase-13, SEQ ID NO: 13, *Bacteriophage* RM378, RNA ligase 1: 
MESMNVKYPVEYLIEHLNSFESPEVAVESLRKEGIMCKNRGDLYMFKYHLGCKFDKIYHLACR
GAILRKTDSGWKVLSYPFDKFFNWGEELQPEIVNYYQTLRYASPLNEKRKAGFMFKLPMKLVEKL
DGTCVVLYYDEGWKIHTLGSIDANGSIVKNGMVTTHMDKTYRELFWETFEKKYPPYLLYHLNSSYC
YIFEMVHPDARVVVPYEEPNIILIGVRSVDPEKGYFEVGPSEEAVRIFNESGGKINLKLPAVLSQEQN
YTLFRANRLQELFEEVTPLFKSLRDGYEVVYEGFVAVQEIAPRVYYRTKIKHPVYLELHRIKTTITPE
KLADLFLENKLDDFVLTPDEQETVMKLKEIYTDMRNQLESSFDTIYKEISEQVSPEENPGEFRKRFA
LRLMDYHDKSWFFARLDGDEEKMQKSEKKLLTERIEKGLFK.
ligase-14, SEQ ID NO: 14, *Citrobacter phage* CkP1, RNA ligase 1: 
MKKLFKNLMALCDEADESKFFYRDDISPSGLKYRIFSYNYASYSDWIRPDALECRGIMFEMID
DKPVRIASRPMEKFFNLNETPFTMNLDLSKAKYMLDKADGSLVSTFLDGNILKFKSKSSIKSEQSYF
STAMLTESRHEALLARLLDLASDGFTANFEYVSPDNRIVLPYQEKQLILLNIRDNDTGEYVDYEDIFK
DGVLRQYLVQRHEITDSNFVEDIRKLTDIEGFVFVMEDGLRFKLKTDWYCALHHTKDSITNNERLFE
SIVNNASDDLKAMFAGDEFAVNKINKFEENYLKYLGESLHLINTTYHELRGRDRKDYAIESQNRANK
SGLGFLFSIIMKMYNGGMDHDTRVKNLSELFMKNYKQFVPKEFI.
ligase-15, SEQ ID NO: 15, *Escherichia phage* JN02, RNA ligase 1: 
MEKLYYNLLSLCKSSSDRKFFYSDDVSPIGKKYRIFSYNFASYSDWLLPDALECRGIMFEMDG
ETPVRIASRPMEKFFNLNENPFTLSINLDDVKYLMTKEDGSLVSTYLDGGTVRFKSKGSIKSDQAVS
ATSILLDIDHKNLADRLLELCNDGFTANFEYVAPTNKIVLTYPEKRLILLNIRDNNTGEYIEYDDIYLDP
VFRKYLVDRFEVPEGDWTSDVKSSTNIEGYVAVMKDGSHFKLKTDWYVALHTTRDSISSPEKLFLA
IVNGASDDLKAMYADDEFSFKKVELFEKAYLDFLDRSFYICLDTYDKHKGKDRKTYAIEAQAVCKGA
QTPWLFGIIMNLYQGGSKEQMMTALESVFIKNHKNFIPEGY.
ligase-16, SEQ ID NO: 16, *Klebsiella phage* KP179, RNA ligase 1: 
MLELYKNLMNLCESSEVAKFFYKDFTGPMDGKFRVFSYHYASYSEWLKPDALECRGIMFEMD
GDTPIRIASRPMEKFFNLNENPLTMGIDISDVEYIMDKADGSLVSSYVDDGYLYLKSKTSLYSDQAR
QASALLNSEEYSSLHQVILELTLDGYTVNMEFVSPNNRVVLAYQEPQLFVLNVRNNTTGEYIKYDD
LYANAKIRPYLINAYGISDPTTWVEGVRELEGVEGYIAVLNTGQRFKVKTEWYSALHHTKDSITSNE
RLFASVVSANSDDLRSLFAGDEYAIKKISAFEQAYLDYLGKSLELCQSFYDEYRGRARKDYAIAAQK
ATVNQRHLFGVIMNMYEGTVDVDKLLKDLERVFLKYWAGYVPKEYEKEIELSEE.
ligase-17, SEQ ID NO: 17, *Vibrio phage* nt-1, RNA ligase 1: 
MNELYNNLMTLAESAEGKFFFADHLSPQGEKFRVFSYHIASYSDWLLPDALEARGIMFQLDEN
DEMIRIVSRPMEKFFNLGENPFTMDLDLTTTVQLMDKADGSLISTYLTGDNFALKSKTSIYSEQAVA
ANRYIKHVDNRDLWEFCDDCAAQNFTVNMEWCAPNNRIVLEYTEPKLIILNIRDNETGQYVSFDDIP
IGALTRIKKWLVDEYDPMTAHVDDFVETLKAKKGIEGMILRLASGQSVKIKTTWYVDLHAQKDSVNS
PKKLVTTILNKNHDDLYALFADDKPTIERIREFDDHVSKKVVESFHAVSQYYTKNRHLSRKDFAIAGQ
KALKPWEFGVAMIAYQNQTVEGVVTMLINAYLKRPELLIPEKYLSEV.
ligase-18, SEQ ID NO: 18, *Vibrio phage* VH7D, RNA ligase 2: 
MSFVKYTSLENSYRQAFVDKCDMLGVKEWVALEKIHGANFSFIVEFKPSTEEVPGEMSVTPA
KRTSTIGANAMGDYDFYGCTSVVEAHIEKMQDISNWLFANDFIKNDETIIVYGELAGKGIQKEVNYG
DKDFWAYDILCPETGEFLDWDVVLKACKFAGVKTTHEIARGTLDELLKIDPLFRSFHTPADVDSENV
AEGFVVKQLKAEKRLHNGSRAILKVKNEKFKEKKNKQGKTPRAKVVLTEEQEKLHAAFSCYLTENR
LRNVLSKIGKVEAKQFGMVSGLFVKDAKDEFERDERDEVAIPRDDWDVIKRSLVNVANEILRKNWL
NIVDGTF.
ligase-19, SEQ ID NO: 19, *Klebsiella phage* KP15, RNA ligase 2: 
MFKKYSSLTNHYEGKFINGVIMNGLTGGVWVAREKIHGANFSFITDDGITVTPAKRTDVVKPAE
DFYGCSAVVAKYSPGIRKMWETLKKTGTYDDLVIQVYGEFAGRGVQKDVDYGEKDFYVFDIRVNG
EFLPDNLCSLISRSHGLKMAPLLGYGTFEEIKELPITFESVVNKANSGIGSDNTVYGEFVYPIMDVEE
GNIAEGFVMKPVSPAFMPNGERVAIKCKTTKFTEKKAKKATRFNAPVSLSEKDKNQLDEFVCYLTE
NRVKNVLSKLDLASITAKDFGRIMGLTVQDAIEEISRNHGPFIEQFEDPAMAKKLFVTEAQNMIRPV
WGKILNHEF.
ligase-20, SEQ ID NO: 20, *Escherichia phage* JN02, RNA ligase 2: 
MFKKYSSLENHYNSKFIEKLYTNGLTTGVWVAREKIHGTNFSLIIERDNVTCAKRTGPILPAEDF
YGYEIVLKKYDKAIKAVQEVMESISTSVPVSYQVFGEFAGGGIQKGVDYGEKDFYVFDIIINTESDDT
YYMSDYEMQDFCNTFGFKMAPMLGRGTFDSLIMIPNDLDSVLAAYNSTASEDLVEANNCVFDANVI
GDNTAEGYVLKPCFPKWLSNGTRVAIKCKNSKFSEKKKSDKPVKTQVPLTEIDKNLLDVLACYVTL
NRVNNVISKIGTVTPKDFGKVMGLTVQDILEETSREGIVLTSSDNPNLVKKELVRMVQDVLRPAWIE
LVS.
ligase-21, SEQ ID NO: 21, *Escherichia phage* JS98, RNA ligase 2: 
MFKKYSSLENHYNSKFIEKLRTNGLTGGEWVAREKIHGTNFSLIIERDAVTCAKRTGPILPAEDF
YGYEIVLKNYADSIKSVQHLIESINYQSYQIYGELAGPGIQKNVDYGDKDFYVFDIRVTKEDGTESVL
TDTLMEAFCIIHKFKVAPCLATGSFEDLIKLPNDFDSVIPDYNFAVDNAGLTIANSTDFIPKVEGKVFT
AEGFVLKPDIPTWLPNGNRVAIKCKNSKFSEKKKSDKPIKAAVVLSQDDMDLMWQFTDYVTVNRIN
NVISKIGEVSKKDFGKVMGLTVQDILEEAAREELELTDAENPVEVKKQLVECVKDTLRAVWIELVS.
ligase-22, SEQ ID NO: 22, *Yersinia phage* fPS-2, RNA ligase 2: 
MFKKYSSLENHYNSKFIEKLYSLGLTGGEWVAREKIHGTNFSLIIERDKVTCAKRTGPILPAEDF
FGYEIILKNYADSIKAVQDIMETSAVVSYQVFGEFAGPGIQKNVDYGDKDFYVFDIIVTTESGGVTYV
DDYMMESFCNSFGFKIAPLLGRGKFEDLIKLPNDLDSVVQDYNFTVDHAGLVDANKCVWNAEAKG
EVFTAEGYVLKPCYPSWLPNRNRVAIKCKNSKFSEKKKSDKPIKAKVELSEADNKLVGILACYVTLN
RVNNVISKIGEIGPKDFGKVMGLTVQDILEETSREGITLTQADNPSLIKKELVKMVQDVLRPAWIELV
S.
ligase-23, SEQ ID NO: 23, *Thermococcus barophilus,* RNA ligase 1: 
MVSLHFKHILLKLGLDKERIEILEMKGGIVEDEFEGLRYLRFKDSAKGLRRGTVVFNESDIILGF
PHIKRVVHLRNGVKRIFKSKPFYVEEKVDGYNVRVAKVGEKILALTRGGFVCPFTTERIGDFINEQF
FKDHPNLILCGEMAGPESPYLVEGPPYVEEDIQFFLFDIQEKRTGRSIPVEERIKLAEEYGIQSVEIF
GLYSYEKIDELYELIERLSKEGREGVVMKSPDMKKIVKYVTPYANVNDIKIGSRIFFDLPHGYFMQRI
KRLAFYIAEKRIRREDFDEYAKALGKALLQPFVESIWDVAAGEMIAEIFTVRVKKIETAYKMVSHFER
MGLNIHIDDIEELGNGYWKITFKRVYDDATKEIRELWNGHAFVD.
ligase-24, SEQ ID NO: 24, *Vibrio phage* ValKK3, RNA ligase 2:

MSFVKYTSLENSYRQAFVDKCDMLGVKEWVALEKIHGANFSFIVEFKPSTEQVPGEMSVTPA
KRTSTIGANAMGDYDFYGCTSVVEAHIEKMQDISNWLFANDFIKNDETIIVYGELAGKGIQKEVNYG
DKDFWAYDILCPETGEFLDWDVVLKACKFAGVKTTHEIARGTLDELLKIDPLFRSFHTPAAVDSENV
AEGFVVKQLKAEKRLHNGSRAILKVKNEKFKEKKNKQGKTPRAKVVLTEEQEKLHAAFSCYLTENR
LRNVLSKIGKVEAKQFGMVSGLFVKDAKDEFERDERDEVAIPRDDWDVIKRSLVNVANEILRKNWL
NIVDGTF.

Identity in the specification refers to "identity" between amino acid sequences or nucleotide sequences, that is, the total of ratios of the amino acid residues or nucleotides of the same type in the amino acid sequences or nucleotide sequences. The identity of the amino acid sequences or nucleotide sequences may be determined with comparison programs such as a Basic Local Alignment Search Tool (BLAST), FASTA, etc.

Active sites, active pockets, active mechanisms, protein structures, etc. of proteins having more than 70%, 75%, 80%, 85%, 90%, 95%, 99% (e.g., more than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, or even more than 99.9%) identity and having the same functions are probably the same as the protein provided by the above sequence).

As used herein, the amino acid residues are abbreviated below: Alanine (Ala; A), Asparagine (Asn; N), Aspartic acid (Asp; D), Arginine (Arg; R), Cysteine (Cys; C), Glutamate (Glu; E), Glutamine (GIn; Q), Glycine (Gly; G), Histidine (His; H), Isoleucine (Ile; I), Leucine (Leu; L), Lysine (Lys; K), Methionine (Met; M), Phenylalanine (Phe; F), Proline (Pro; P), Serine (Ser; S), Threonine (Thr; T), Tryptophan (Trp; W), Tyrosine (Tyr; Y), and Valine (Val; V).

Generally, according to rules such as substitution, replacement, etc., amino acids with similar properties have a similar effect when they are substituted for each other. For example, in the protein, conservative amino acid replacements may occur. "Conservative amino acid replacement" includes, but is not limited to the followings.

Hydrophobic amino acids (Ala, Cys, Gly, Pro, Met, Val, Ile, and Leu) are substituted by other hydrophobic amino acids.

Hydrophobic amino acids with bulky side chains (Phe, Tyr, and Trp) are substituted by other hydrophobic amino acids with bulky side chains.

Amino acids with positively charged side chains (Arg, His, and Lys) are substituted by other amino acids with positively charged side chains.

Amino acids with polarized and uncharged side chains (Ser, Thr, Asn, and GIn) are substituted by other amino acids with polarized and uncharged side chains.

Person skilled in the art may also make conservative substitutions of the amino acids according to amino acid substitution rules known to person skilled in the art, such as a "blosum62 scoring matrix" in the prior art.

In a preferred embodiment, the number of the substrates is two or more.

In a preferred embodiment, the gRNA has a length of 67-142 nt.

In a preferred embodiment, the gRNA has a length of 91-104 or 130-146 nt.

The gRNA has a length of 67-142 nt, including, but not limited to, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, or 142 nt.

The gRNA includes crRNA and tracrRNA, where the crRNA has a length of 17-24 nt, preferably 20 nt; and the tracrRNA has a length of 50-121 nt, preferably 80 nt.

The number of the substrates includes, but is not limited to, 2, 3, 4, 5, or more. The gRNA can be prepared with the above method.

In a preferred embodiment, the sequence of the gRNA includes a nucleotide sequence shown in any one of SEQ ID NO: 25, SEQ ID NO: 26, SEQ ID NO: 27, SEQ ID NO: 28, SEQ ID NO: 41, SEQ ID NO: 42 or SEQ ID NO: 43.

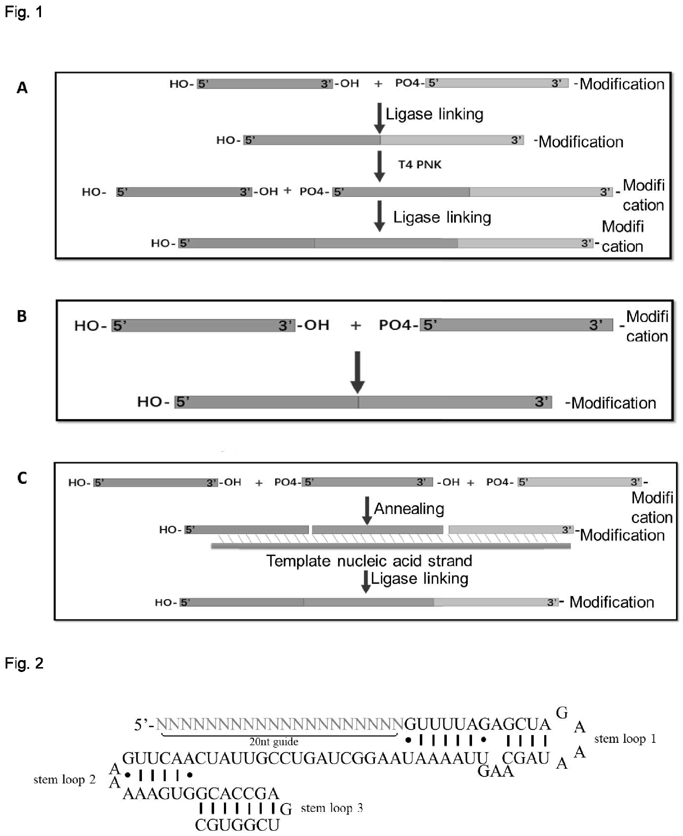

In the embodiments of the present application, the synthesized gRNA is a 100nt sequence derived from *Streptococcus pyogenes* and used in a commonly-used CRISPR-Cas9 system and a 142nt sequence derived from *Neisseria meningitidis* and used in the CRISPR-Cas9 system. The gRNA of the CRISPR-Cas9 systems from different sources has similar secondary structure shown in Fig. 2 (Fuchsbauer et. al., 2019; Hirano et. al., 2016; Hirano et. al., 2019; Hsu et. al., 2013; Ran et. al., 2015; Yamada et. al., 2017). The gRNA suitable for other CRISPR-Cas9 systems can also be synthesized with the above method for gRNA.

The gRNA sequence includes a variable sequence of 15-25 nt. In the above sequence, bases (nucleotides) N are each independently selected from any one of A, U, G, and C. Flexible changes may be made according to crRNA required for gene editing, thereby achieving target editing for different target fragments. Other bases (non-N nucleotides) on the above gRNA sequence may also be designed and changed according to the preferences of CRISPR and Cas proteins.

In a preferred embodiment, the substrates include:
SEQ ID NO:29 or a nucleotide sequence consisting of 15-25 N nucleotides; and nucleotide sequences shown in SEQ ID NO:30 and SEQ ID NO:31; or a nucleotide sequence as shown in SEQ ID NO: 32 or a sequence comprising the nucleotide sequence consisting of 15-25 N nucleotides and a nucleotide sequence as shown in SEQ ID NO: 36, and a nucleotide sequence as shown in SEQ ID NO: 33; wherein the nucleotide sequence consisting of 15-25 N nucleotides is in the 5' direction of the nucleotide sequence as shown in SEQ ID NO: 36; or a nucleotide sequence as shown in SEQ ID NO: 44 or a sequence comprising the nucleotide sequence consisting of 15-25 N nucleotides and a nucleotide sequence as shown in SEQ ID NO: 47, and nucleotide sequences as shown in SEQ ID NO: 45 and SEQ ID NO: 46; wherein the nucleotide sequence consisting of 15-25 N nucleotides is in the 5' direction of the nucleotide sequence as shown in SEQ ID NO: 36; or a nucleotide sequence as shown in SEQ ID NO: 48 or a sequence comprising the nucleotide sequence consisting of 15 to 25 N nucleotides and a nucleotide sequence as shown in SEQ ID NO: 50, and a nucleotide sequence as shown in SEQ ID NO: 49; wherein the nucleotide sequence consisting of 15-25 N nucleotides is in the 5' direction of the nucleotide sequence as shown in SEQ ID NO: 50.

The variable sequence (N nucleotides) in the sequences of the above substrates can be flexibly changed, as described above.

In a preferred embodiment, when the number of the substrates is 2, the method includes: the substrates and the RNA ligase are mixed, where the substrates include a first substrate and a second substrate, the RNA ligase links a phosphate group at the 5' end of the first substrate and a hydroxyl group at the 3' end of the second substrate to form the phosphodiester bond, thereby obtaining the gRNA.

The lengths of the first substrate and second substrate may be flexibly changed, and include, but are not limited to, 5, 10, 15, 20, 25, 30, 35, 40, 45, 50, 55, 60, 65, 70, 75, 80, 85, 90, 95, 100, 105, 110, 115, 120, 125, 130, 135, or 137 nt, ensuring that the sum of the lengths of the first substrate and second substrate is 67-142 nt.

In a preferred embodiment, the 5' end of the second substrate is a 5' end protecting group, and the 3' end of the first substrate is a 3' end protecting group.

In a preferred embodiment, when the number of the substrates is 3, the method includes: a first substrate, a second substrate, and the RNA ligase are mixed, where the 5' end of the first substrate is a phosphate group, the 3' end of the first substrate is a 3' end protecting group, and the 5' end and 3' end of the second substrate are hydroxyl groups; the phosphate group at the 5' end of the first substrate and the hydroxyl group at the 3' end of the second substrate are ligated to form the phosphodiester bond with the RNA ligase, so as to obtain an intermediate product of which the 5' end is a hydroxyl group; 5' phosphorylation is performed on the intermediate product to obtain a 5' phosphorylated intermediate product; and the 5' phosphorylated intermediate product, a third substrate and the RNA ligase are mixed, and the phosphate group at the 5' end of the 5' phosphorylated intermediate product and the hydroxyl group at the 3' end of the third substrate are ligated to form the phosphodiester bond with the RNA ligase, so as to obtain the gRNA, where the 5' end of the third substrate is a 5' end protecting group.

In a preferred embodiment, by repeatedly performing ligation and 5' phosphorylation and sequentially ligating the substrates via the method, four or more substrates are subjected to multiple rounds of ligation and 5' phosphorylation. Two substrates are used in each ligation step, and the substrates are sequentially ligated to obtain the gRNA.

With the above method, the plurality of substrates can be gradually ligated to form the gRNA through fragmented ligating, such that the plurality of substrates are prevented from mixing to generate unordered ligating between the substrates. The 5' end of the intermediate product is initially a hydroxyl group, and it can ensure that self-ligating does not occur in the substrates when the intermediate product is prepared. After the intermediate product is obtained through preparation, 5' phosphorylation is performed on the intermediate product with, including but not limited to, polynucleotide kinase, thereby realizing next ligating. After 5' phosphorylation and before RNA ligating, a deactivation treatment needs to be performed on 5' phosphorylase.

In a preferred embodiment, a template nucleic acid strand is used to guide the ligating of the substrates; the template nucleic acid strand has at least 3 base specific bindings with each of different substrates, the bases of the substrates specifically binding to the template nucleic acid chain are adjacent and form a nick, forming a double-stranded nucleotide structure containing the nick, bases adjacent to two ends of the nick are a 5' end and a 3' end of different substrates, the 5' end is a 5' phosphate, and the 3' end is a hydroxyl group; the 5' phosphate and the hydroxyl group at upstream and downstream of the nick are ligated to form the phosphodiester bond with the RNA ligase, so as to obtain a double-stranded nucleotide structure; and the template nucleic acid strand of the double-stranded nucleotide structure is digested with a DNase to obtain the gRNA.

In a preferred embodiment, a sequence of the template nucleic acid strand includes any one of the nucleotide sequences as shown in SEQ ID NO: 37, SEQ ID NO: 39, SEQ ID NO: 40, or SEQ ID NO: 51.

In a preferred embodiment, after mixing the template nucleic acid strand with different substrates, high-temperature incubation is performed, and then slow cooling and annealing or constant-temperature incubation is performed, so as to obtain the double-stranded nucleic acid structure containing the nick. A temperature of the constant temperature incubation is 4-37 °C, and a time of the constant temperature incubation is ≥10 min; and performing high-temperature incubation and then performing slow cooling and annealing includes: incubation is performed at 95 °C for 2 min, the temperature is reduced to 12 °C at a rate of 1.2 °C/min, incubation is performed at 12 °C for 10 min, and then the temperature is reduced to 4 °C.

In a preferred embodiment, the 3' end of the substrate at the 3' end of the gRNA is a 3' end protecting group, and the 5' end of the substrate at the 5' end of the gRNA is a 5' end protecting group.

The above template nucleic acid strand is a single DNA strand consisting of deoxyribonucleotide. All the substrates can achieve complementary base pairing with the template nucleic acid strand. Therefore, in the above method, the correct arrangement of the substrates can be guided with the template nucleic acid strand, thereby preventing the substrates from ligating incorrectly. RNA ligating is guided with the template nucleic acid strand. The substrates first bind to the template nucleic acid strand. After the substrates specifically bind to the template nucleic acid strand, a nick is generated between the substrates, thereby obtaining the double-stranded nucleotide structure with the nick. The two ends of the nick respectively are the 5' end and 3' end of different substrates, where the 5' end is the 5' phosphate, and the 3' end is the hydroxyl group. The 5' phosphate and the 3' hydroxyl group on the two ends of the nick can be ligated with the RNA ligase, so as to form the phosphodiester bond, thereby obtaining a double-stranded nucleotide structure without the nick. Then, the template nucleic acid strand on the double-stranded nucleotide structure is digested with a DNase to obtain the single-stranded gRNA. The single-stranded gRNA internally has bases that can be complementarily paired with each other. Complementary pairing can be automatically performed to form the secondary structure, thereby obtaining the gRNA containing the secondary structure.

For 3, 4, or even more substrates, considering the ligating efficiency of each step, every time a substrate is added during multi-step ligating, the yield decreases. However, the ligating (splinted ligation) of the substrates is guided with the template nucleic acid strand, and then a one-step reaction may be performed with essentially unchanged yield.

In a preferred embodiment, the secondary structure of the gRNA is formed by the substrates themselves or by spontaneous complementary base pairing between the substrates.

The above complementary base pairing automatically occurring may be automatically formed at room temperature or 4 °C, and slow cooling and annealing after high-temperature incubation is more conductive to the formation of the secondary structure.

In a preferred embodiment, the 3' end protecting group includes modification groups, such as a N-acetylgalactosamine group (GalNac), a phosphate group, -O-NH₂, azido (-C-N = N⁺ = N⁻, 3'-O-azidomethyl), cyanoethyl (-C-CN, 3'-O-2-cyanoethyl), allyl (-C-C = C, 3'-O-allyl), 2-nitrobenzyl (3'-O-2-nitrobenzyl), etc., that can prevent the formation of a phosphoester bond; and the 5' end protecting group includes modification groups, such as hydroxyl group, hydrogen or methyl group, etc., that can prevent the formation of the phosphoester bond.

It is sufficient that the above 3' end protecting group is not the hydroxyl group, and the above 5' end protecting group is not the phosphate group. For the substrate with the 3' end hydroxyl group, the 5' end is not the phosphate group; and for the substrate with the 5' end phosphate group, the 3' end is not the hydroxyl group, preventing cyclization and self-ligating of the substrates, thus affecting the yield and product purity of the method. Non-natural RNA containing modifications can also be obtained through preparation using this method.

In a preferred embodiment, the substrate includes one or more non-natural nucleotides, and the non-natural nucleotide includes: a ribonucleotide having one or more of a ribose 2' position modification, a ribose backbone modification, a base modification or a phosphate backbone modification; the ribose position 2' modification includes a 2'-methoxy modification, a 2'-fluoro modification, a 2'-hydrogen modification, a 2'-methoxyethyl modification, a 2'-FANA modification, a locked nucleic acid modification, or a hexitol nucleic acid modification; the ribose backbone modification includes replacing a ribose in the nucleotide with ribuloNA, TNA, tPhoNA, or dXNA; the base modification includes a deazaadenine C7 modification, a deazaguanosine C7 modification, a cytosine C5 modification or a uridine C5 modification; and the phosphate backbone modification includes a PS modification.

Xeno-nucleic acids (XNA) are a class of nucleic acid molecules having non-natural backbones or nucleic acid bases. Non-natural ribonucleotide is a ribonucleotide having a non-natural backbone or nucleic acid base. The above substrate contains the XNA, and the gRNA prepared through ligating contains the non-natural nucleotide, belonging to the non-natural RNA (non-natural gRNA).

The locked nucleic acid modification is 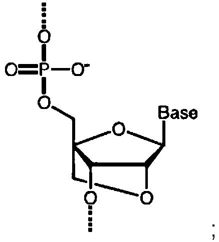 the hexitol nucleic acid modification is 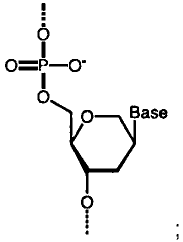 2' methoxyethyl modification is 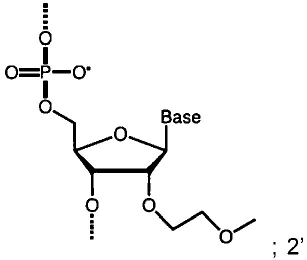 methoxy modification is 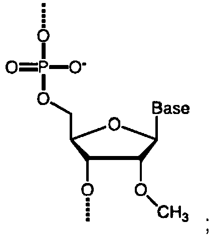 2' fluoro modification is 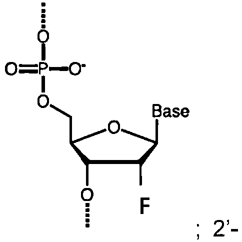 FANA is 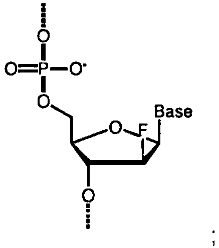 ribuloNA is 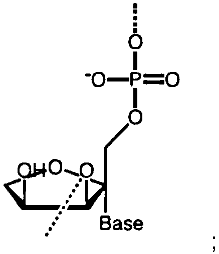 TNA is 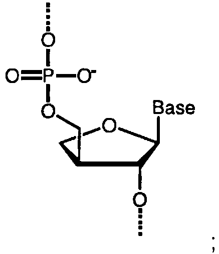 tPhoNA is 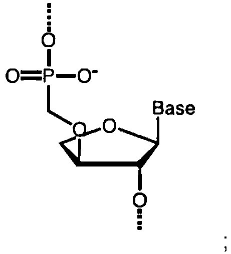 the dXNA is 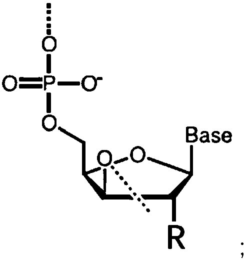 and PS modification is 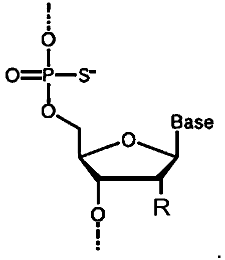 Bases in the above structures all represent bases. For the above modification of non-natural nucleotides, refer to literature: Duffy K, Arangundy-Franklin S, Holliger P. Modified nucleic acids: replication, evolution, and next-generation therapeutics [J].BMC Biology, 2020, 18(1): 112. In the non-natural nucleotide, the 2' modification increases 2' steric hindrance, such as methoxy, or changes the property of the 2' group, such as fluoro. The method disclosed in the present application can link 2' modified nucleic acids, such that the ligating of the natural nucleic acids having the 2' hydroxyl groups can also be realized with the above method, thereby obtaining the natural gRNA.

In a second typical implementation of the present application, gRNA is provided. The gRNA includes the gRNA obtained through preparation using the above method.

The beneficial effects of the present application are further described in detail below with reference to specific embodiments. Reaction schematic diagrams of Embodiments 1, 2, and 3 are shown in Fig. 1.

### Embodiment 1

An ssRNA ligase ligated three fragments of single-stranded RNA to form gRNA of 100 nt, and a reaction schematic diagram was shown in A in Fig. 1.

The sequence of the gRNA included, but was not limited to, a sequence shown in SEQ ID NO: 25, SEQ ID NO: 26, or SEQ ID NO: 27. The gRNA sequence included a variable sequence of 15-25 nt, that is, the number of N at the 5' end of the gRNA might be optionally selected as any integer among 15-25, including 15, 16, 17, 18, 19, 20, 21, 22, 23, 24, or 25.
SEQ ID NO: 25: 
NNNNNNNNNNNNNNNNGUUUUAGAGCUAGAAAUAGCAAGUUAAAAUAAGGCUAGUCCGU
UAUCAACUUGAAAAAGUGGCACCGAGUCGGUGC;
SEQ ID NO: 26: 
NNNNNNNNNNNNNNNNNNNNGUUUUAGAGCUAGAAAUAGCAAGUUAAAAUAAGGCUAG
UCCGUUAUCAACUUGAAAAAGUGGCACCGAGUCGGUGC; or
SEQ ID NO: 27:

NNNNNNNNNNNNNNNNNNNNGUUUUAGAGCUAGAAAUAGCAAGUUAAAAUAAG
GCUAGUCCGUUAUCAACUUGAAAAAGUGGCACCGAGUCGGUGC.

The nucleotides N were nucleotides that was flexibly designed according to a CRISPR target fragment, and might be respectively selected as A, U, G, or C.

A target synthetic sequence 1: SEQ ID NO: 28 in this embodiment:

GACUGAUAUACACAGGCCGAUGUGGUUUUAGAGCUAGAAAUAGCAAGUUAAAAUAAGG
CUAGUCCGUUAUCAACUUGAAAAAGUGGCACCGAGUCGGUGC.

Positions 2' of all ribonucleotides in the above sequence shown in SEQ ID NO: 28 were methoxy modifications.

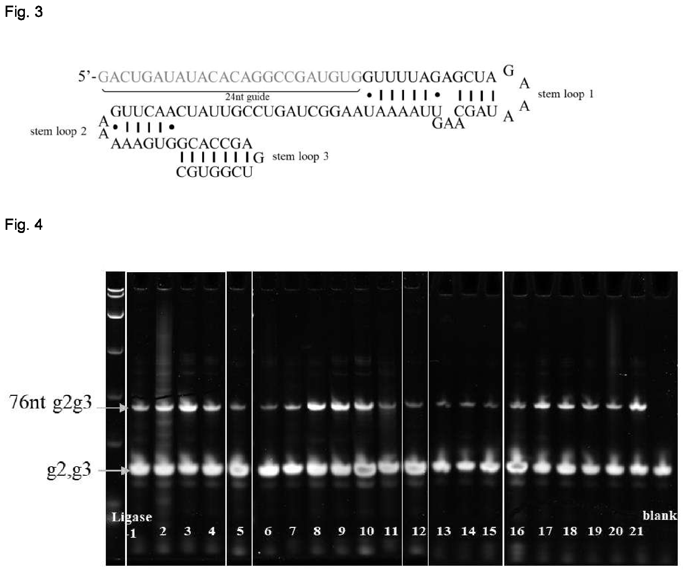

A secondary structure of SEQ ID NO: 26 was shown in Fig. 2, and a secondary structure of SEQ ID NO: 28 was shown in Fig. 3.

Three steam loops in Fig. 2 and Fig. 3 constituted a stem-loop structure of gRNA. The stem-loop structure included a stem-loop structure complementary region and a stem-loop structure loop region, which were formed through complementary base pairing. Guides of 20 nt and 24 nt were variable regions consisting of the nucleotides N.

Step 1(S1): The substrate fragments g2 and g3 were ligated with an RNA ligase.

A ligating reaction was performed with the RNA ligase; a reaction system included 50 mM Tris-HCl (pH 7.5), 10 mM MgCl₂, 1 mM DTT, 100 µM g2, 100 µM g3, 200 µM ATP, and 0.2 mg/mL RNA ligase; a reaction temperature was 37 °C, and a reaction time was 16 h; and after the reaction completed, incubation was performed for 5 min at 80 °C, and then the reaction was terminated to obtain an intermediate product g2g3.

The reaction system was detected with urea-denatured polyacrylamide gel electrophoresis. Gel electrophoresis detection results were shown in Fig. 4, blank was a negative control group without adding the RNA ligase. Compared with the blank, new RNA bands were detected in the reaction system of the RNA ligases ligase-1, ligase-2, ligase-3, ligase-4, ligase-5, ligase-6, ligase-7, ligase-8, ligase-9, ligase-10, ligase-11, ligase-12, ligase-13, ligase-14, ligase-15, ligase-16, ligase-17, ligase-18, ligase-19, ligase-20, and ligase-21 between 50 nt and 80 nt. The molecular weight of the new band detected through liquid chromatography-mass spectrometry analysis was consistent with the theoretical molecular weight of the ligation product of g2 and g3.

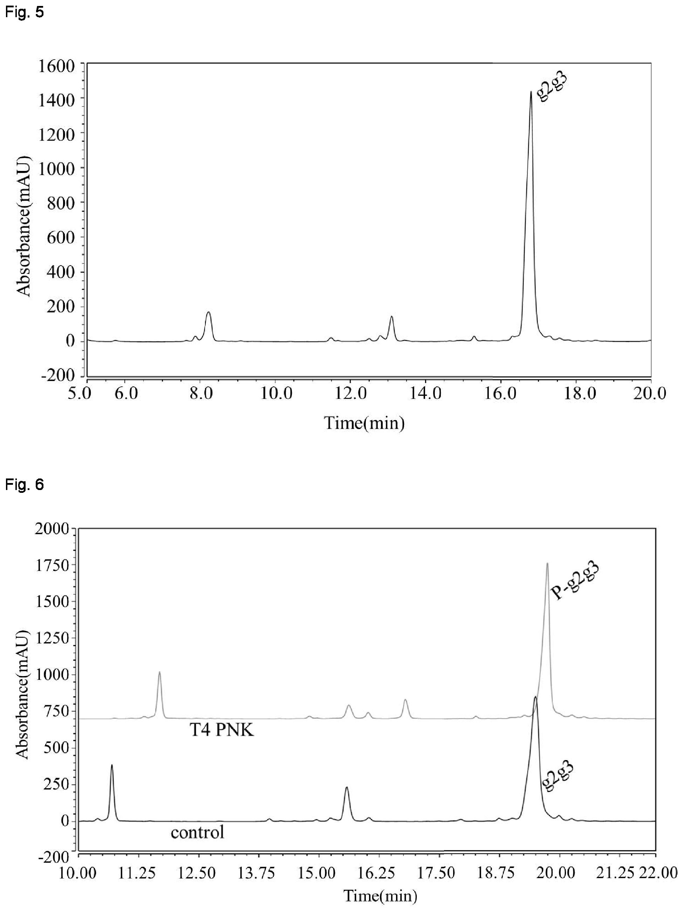

The reaction system was detected using liquid chromatography (UPLC). Results were shown in Fig. 5. The catalytic activity of the RNA ligase through UPLC detection was shown in the table below (Table 1).

**Table 1**

| RNA ligase name | Sequence No. | Purity of g2g3 product system |
|---|---|---|
| ligase-1 | SEQ ID NO: 1 | +++ |
| ligase-2 | SEQ ID NO: 2 | ++++ |
| ligase-3 | SEQ ID NO: 3 | +++++ |
| ligase-4 | SEQ ID NO: 4 | ++++ |
| ligase-5 | SEQ ID NO: 5 | ++ |
| ligase-6 | SEQ ID NO: 6 | ++ |
| ligase-7 | SEQ ID NO: 7 | +++ |
| ligase-8 | SEQ ID NO: 8 | +++++ |
| ligase-9 | SEQ ID NO: 9 | +++++ |
| ligase-10 | SEQ ID NO: 10 | +++++ |
| ligase-11 | SEQ ID NO: 11 | ++ |
| ligase-12 | SEQ ID NO: 12 | ++ |
| ligase-13 | SEQ ID NO: 13 | ++ |
| ligase-14 | SEQ ID NO: 14 | ++ |
| ligase-15 | SEQ ID NO: 15 | ++ |
| ligase-16 | SEQ ID NO: 16 | ++ |
| ligase-17 | SEQ ID NO: 17 | ++++ |
| ligase-18 | SEQ ID NO: 18 | ++++ |
| ligase-19 | SEQ ID NO: 19 | ++++ |
| ligase-20 | SEQ ID NO: 20 | +++ |
| ligase-21 | SEQ ID NO: 21 | ++++ |

| | | |
|---|---|---|
| Note: "++" indicated 20-40% purity of the product system; "+++" indicated 40-60% (excluding an endpoint value of 40%) purity of the product system; "+++" indicated 60-80% (excluding an endpoint value of 60%) purity of the product system; and "++++" indicated 80-100% (excluding an endpoint value of 80%) purity of the product system. In the present application, "the purity of the product system" indicated that in the reaction system, a ratio of a target product to a total component (target product + remaining substrate) in the reaction system might be calculated by the area under the curve of different components in liquid chromatography results. | | |

The ligation reaction of g2 and g3 was performed under a high substrate concentration condition, and a substrate concentration was 500 or 700 µM. The catalytic activity of the RNA ligase through UPLC analysis was shown in Table 2.

**Table 2**

| Enzyme | Substrate concentration/µM | Product system purity in g2+g3 reaction system/% |
|---|---|---|
| ligase-3 | 100 | 84.77 |
| | 500 | 9.16 |
| | 700 | 4.37 |
| ligase-9 | 100 | 83.84 |
| | 500 | 49.78 |
| | 700 | 50.82 |
| ligase-10 | 100 | 81.75 |
| | 500 | 22.48 |
| | 700 | 17.88 |
| Ligase-21 | 100 | 76.60 |
| | 500 | 54.18 |
| | 700 | 18.56 |

In Table 2, the reaction system with the substrate concentration of 100 µM included 50 mM Tris-HCl (pH 7.5), 10 mM MgCl₂, 1 mM DTT, 100 µM g2, 100 µM g3, 200 µM ATP, and 0.2 mg/mL RNA ligase; a reaction temperature was 37 °C, and a reaction time was 16 h; and after the reaction completed, incubation was performed for 5 min at 80 °C, and then the reaction was terminated.

The reaction system with the substrate concentration of 500 µM included 50 mM Tris-HCl (pH 7.5), 10 mM MgCl₂, 1 mM DTT, 500 µM g2, 500 µM g3, 1000 µM ATP, and 0.2 mg/mL RNA ligase; a reaction temperature was 37 °C, and a reaction time was 16 h; and after the reaction completed, incubation was performed for 5 min at 80 °C, and then the reaction was terminated.

The reaction system with the substrate concentration of 700 µM included 50 mM Tris-HCl (pH 7.5), 10 mM MgCl₂, 1 mM DTT, 700 µM g2, 700 µM g3, 1400 µM ATP, and 0.2 mg/mL RNA ligase; a reaction temperature was 37 °C, and a reaction time was 16 h; and after the reaction completed, incubation was performed for 5 min at 80 °C, and then the reaction was terminated.

Step 2(S2): 5' phosphorylation was performed on the 76nt intermediate product g2g3.

Oligonucleotide 5' phosphorylation was performed with T4 Polynucleotide Kinase (T4 PNK, Vazyme, N102-01). In the system in S1, the T4 PNK with a final concentration of 0.25 U/µL, 1 mM ATP, and a T4 PNK Buffer with 10X of a corresponding volume were added. The reaction was performed for more than 3 h at 37 °C. P-g2g3 (g2g3 with 5' end phosphorylated) was prepared after purification.

The phosphorylated product was analyzed through UPLC. UPLC results were shown in Fig. 6. The T4 PNK was a sample prepared in S2, and control was a negative control sample without T4 PNK. For the system added with the T4 PNK, a g2g3 product peak disappeared, and a new peak appeared on its right side. Through liquid chromatography-mass spectrometry identification, the molecular weight of the new peak was the same as the theoretical molecular weight of the P-g2g3.

Step 3(S3): g1 and P-g2g3 fragments were ligated with the RNA ligase.

The reaction system including 50 mM Tris-HCl (pH 7.5), 10 mM MgCl₂, 1 mM DTT, 20 µM g1, 20 µM P-g2g3, 40 µM ATP, and 0.2 mg/mL RNA ligase was prepared; a reaction temperature was 37 °C, and a reaction time was 16 h; and after the reaction completed, incubation was performed for 5 min at 80 °C, and then the reaction was terminated to obtain a product gRNA.

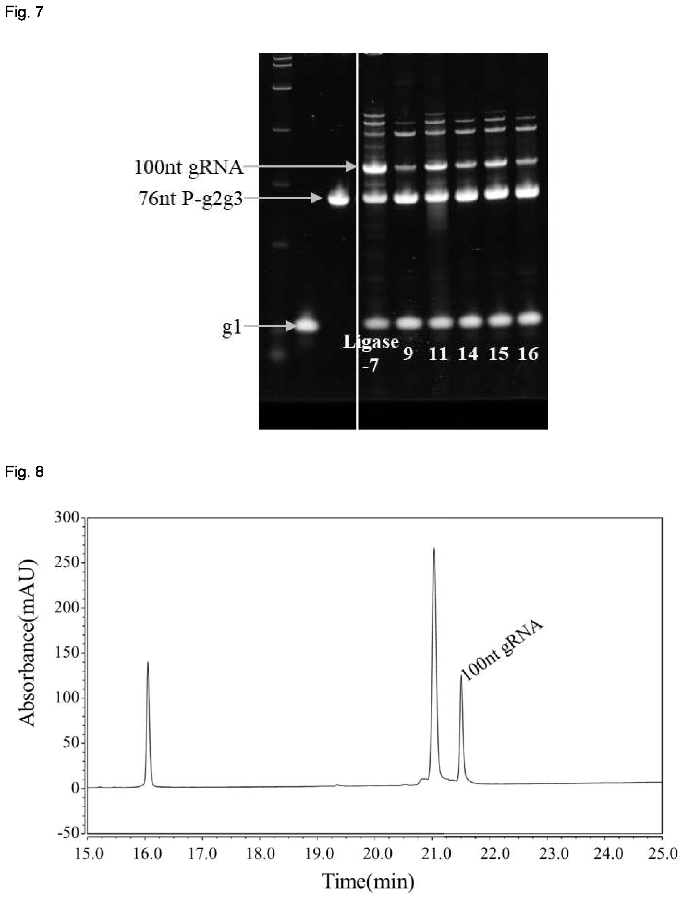

Partial reaction system was detected using urea-denatured polyacrylamide gel electrophoresis. Gel electrophoresis detection results were shown in Fig. 7. A new RNA band was detected 80 nt and 150 nt on a RNA Marker. The molecular weight of the new band detected through liquid chromatography-mass spectrometry analysis was consistent with the theoretical molecular weight of the g1-P-g2g3 ligating product gRNA.

The reaction system was detected using liquid chromatography (UPLC). Results were shown in Fig. 8. The catalytic activity of the RNA ligase through UPLC detection was shown in the table below (Table 3).

**Table 3**

| RNA ligase name | Sequence No. | Purity of 100nt gRNA product system |
|---|---|---|
| ligase-1 | SEQ ID NO: 1 | +++ |
| ligase-2 | SEQ ID NO: 2 | + |
| ligase-3 | SEQ ID NO: 3 | + |
| ligase-4 | SEQ ID NO: 4 | ++ |
| ligase-5 | SEQ ID NO: 5 | + |
| ligase-6 | SEQ ID NO: 6 | ++ |
| ligase-7 | SEQ ID NO: 7 | +++ |
| ligase-8 | SEQ ID NO: 8 | +++ |
| ligase-9 | SEQ ID NO: 9 | ++ |
| ligase-10 | SEQ ID NO: 10 | ++ |
| ligase-11 | SEQ ID NO: 11 | ++++ |
| ligase-12 | SEQ ID NO: 12 | ++ |
| ligase-13 | SEQ ID NO: 13 | +++ |
| ligase-14 | SEQ ID NO: 14 | ++ |
| ligase-15 | SEQ ID NO: 15 | ++++ |
| ligase-16 | SEQ ID NO: 16 | +++ |
| ligase-17 | SEQ ID NO: 17 | ++ |
| ligase-18 | SEQ ID NO: 18 | ++ |
| ligase-19 | SEQ ID NO: 19 | +++ |
| ligase-20 | SEQ ID NO: 20 | ++ |
| ligase-21 | SEQ ID NO: 21 | ++ |

| | | |
|---|---|---|
| Note: "+" indicated 0-5% (excluding an endpoint value of 0%) purity of the product system; "++" indicated 5-10% (excluding an endpoint value of 5%) purity of the product system; "+++" indicated 10-15% (excluding an endpoint value of 10%) purity of the product system; and "++++" indicated 15-20% (excluding an endpoint value of 15%) purity of the product system. In the present application, "the purity of the product system" indicated that in the reaction system, a ratio of a target product to a total component (target product + remaining substrate) in the reaction system might be calculated by the area under the curve of different components in liquid chromatography results. | | |

Sequences of g1, g2, and g3 were shown in Table 4.

**Table 4**

| Serial number | Sequence (5'->3') | Base number | 5' end modification | 3' end modification |
|---|---|---|---|---|
| g1 (SEQ ID NO: 29) | GmAmCmUmGmAmUmAmUmAmCmAm CmAmGmGmCmCmGmAmUmGmUmGm | 24nt | Hydroxyl group | Hydroxyl group |
| g2 (SEQ ID NO: 30) | GmUmUmUmUmAmGmAmGmCmUmAm GmAmAmAmUmAmGmCmAmAmGmUm UmAmAmAmAmUmAmAmGmGmCmUm | 36nt | Hydroxyl group | Hydroxyl group |
| g3 (SEQ ID NO: 31) | AmGmUmCmCmGmUmUmAmUmCmAm AmCmUmUmGmAmAmAmAmAmGmUm GmGmCmAmCmCmGmAmGmUmCmGm GmUmGmCm | 40nt | Phosphate group | Modification |

The m after A, C, G, or U represented the 2' methoxy modification for the ribonucleotide; g1 contained 24 bases that was designed according to the CRISPR target fragment; and corresponding substrate sequences included variable sequences consisting of 24 (optionally 15-25) nucleotides.

### Embodiment 2

An RNA ligase ligated two fragments of 50nt single-stranded RNA to form gRNA of 100 nt, and a reaction schematic diagram was shown in B in Fig. 1.

A target synthetic sequence 1 in this embodiment: SEQ ID NO: 28:

GACUGAUAUACACAGGCCGAUGUGGUUUUAGAGCUAGAAAUAGCAAGUUAAAAUAAGG
CUAGUCCGUUAUCAACUUGAAAAAGUGGCACCGAGUCGGUGC.

Positions 2' of all ribonucleotides in the above sequence were methoxy modifications.

A g4 and g5 ligating reaction was performed with the RNA ligase; a reaction system included 50 mM Tris-HCl (pH 7.5), 10 mM MgCl₂, 1 mM DTT, 100 µM g4, 100 µM g5, 200 µM ATP, and 0.2 mg/mL RNA ligase; a reaction temperature was 37 °C, and a reaction time was 16 h; and after the reaction completed, incubation was performed for 5 min at 80 °C, and then the reaction was terminated to obtain a gRNA product.

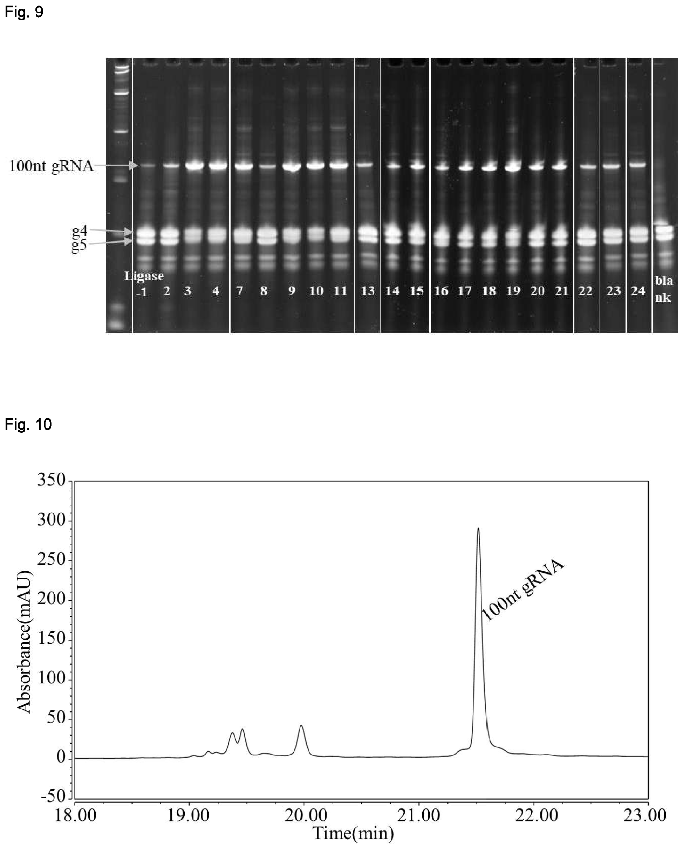

The reaction system was detected with urea-denatured polyacrylamide gel electrophoresis. Gel electrophoresis detection results were shown in Fig. 9, blank was a negative control group without adding the RNA ligase. Compared with the blank, new RNA bands were detected in the reaction system of the RNA ligases ligase-1, ligase-2, ligase-3, ligase-4, ligase-7, ligase-8, ligase-9, ligase-10, ligase-11, ligase-13, ligase-14, ligase-15, ligase-16, ligase-17, ligase-18, ligase-19, ligase-20, ligase-21, ligase-22, ligase-23, and ligase-24 between 80 nt and 150 nt on a RNA Marker. The molecular weight of the new band detected through liquid chromatography-mass spectrometry analysis was consistent with the theoretical molecular weight of the gRNA product.

The reaction system was detected using liquid chromatography (UPLC). Results were shown in Fig. 10. The catalytic activity of the RNA ligase through UPLC detection was shown in the table below (Table 5).

**Table 5**

| RNA ligase name | Sequence No. | Purity of 100nt gRNA product system |
|---|---|---|
| ligase-1 | SEQ ID NO: 1 | + |
| ligase-2 | SEQ ID NO: 2 | ++ |
| ligase-3 | SEQ ID NO: 3 | ++++ |
| ligase-4 | SEQ ID NO: 4 | ++++ |
| ligase-7 | SEQ ID NO: 7 | ++++ |
| ligase-8 | SEQ ID NO: 8 | ++ |
| ligase-9 | SEQ ID NO: 9 | +++++ |
| ligase-10 | SEQ ID NO: 10 | +++++ |
| ligase-11 | SEQ ID NO: 11 | +++++ |
| ligase-13 | SEQ ID NO: 13 | ++ |
| ligase-14 | SEQ ID NO: 14 | +++ |
| ligase-15 | SEQ ID NO: 15 | ++++ |
| ligase-16 | SEQ ID NO: 16 | +++ |
| ligase-17 | SEQ ID NO: 17 | ++++ |
| ligase-18 | SEQ ID NO: 18 | ++++ |
| ligase-19 | SEQ ID NO: 19 | +++++ |
| ligase-20 | SEQ ID NO: 20 | +++ |
| ligase-21 | SEQ ID NO: 21 | ++++ |
| ligase-22 | SEQ ID NO: 22 | ++ |
| ligase-23 | SEQ ID NO: 23 | ++ |
| ligase-24 | SEQ ID NO: 24 | +++ |

| | | |
|---|---|---|
| Note: "+" indicated 0-10% (excluding an endpoint value of 0%) purity of the product system; "++" indicated 10-20% (excluding an endpoint value of 10%) purity of the product system; "+++" indicated 20-30% (excluding an endpoint value of 20%) purity of the product system; "++++" indicated 30-40% (excluding an endpoint value of 30%) purity of the product system; and "+++++" indicated 40-50% (excluding an endpoint value of 40%) purity of the product system. In the present application, "the purity of the product system" indicated that in the reaction system, a ratio of a target product to a total component (target product + remaining substrate) in the reaction system might be calculated by the area under the curve of different components in liquid chromatography results. | | |

Sequences of g4, and g5 were shown in Table 6.

**Table 6**

| Serial number | Sequence (5'->3') | Base number | 5' end modification | 3' end modification |
|---|---|---|---|---|
| g4 (SEQ ID NO: | GmAmCmUmGmAmUmAmUmAmCmAmCm AmGmGmCmCmGmAmUmGmUmGmGmUm | 50nt | Hydroxyl group | Hydroxyl group |
| 32) | UmUmUmAmGmAmGmCmUmAmGmAmAm AmUmAmGmCmAmAmGmUmUmAm | | | |
| g5 (SEQ ID NO: 33) | AmAmAmUmAmAmGmGmCmUmAmGmUm CmCmGmUmUmAmUmCmAmAmCmUmUm GmAmAmAmAmAmGmUmGmGmCmAmCm CmGmAmGmUmCmGmGmUmGmCm | 50nt | Phosphate group | Modification |

The m after A, C, G, or U represented the 2' methoxy modification for the ribonucleotide; g4 contained 24 nucleotides that was designed according to the CRISPR target fragment; and corresponding substrate sequences included sequences containing the variable sequence with 15-25 nt. The number of N at the 5' end of the substrate sequence might be optionally selected as any integer among 15-25, including 15, 16, 17, 18, 19, 20, 21, 22, 23, 24, or 25, including but not limited to sequence shown in SEQ ID NO 34 or SEQ ID NO 35. That is, g4 was a sequence that was formed by ligating the variable sequence with 15-25 nt and a sequence shown in SEQ ID NO: 36, and the variable sequence was located in the 5' direction of the SEQ ID NO: 36.
NNNNNNNNNNNNNNNGUUUUAGAGCUAGAAAUAGCAAGUUA (SEQ ID NO;34),
NNNNNNNNNNNNNNNNNNNNNNNNNGUUUUAGAGCUAGAAAUAGCAAGUUA (SEQ ID NO; 35),
5'-GUUUUAGAGCUAGAAAUAGCAAGUUA-3'(SEQ ID NO: 36).

The g4+g5 ligating reaction was performed under a high substrate concentration condition. The catalytic activity of the RNA ligase through UPLC analysis was shown in Table 7.

**Table 7**

| Enzyme | Substrate concentration/µm | Product system purity in g4+g5 reaction system/% |
|---|---|---|
| ligase-3 | 100 | 23.76 |
| | 300 | 2.08 |
| ligase-9 | 100 | 50.09 |
| | 300 | 12.44 |
| ligase-19 | 100 | 38.73 |
| | 300 | 6.19 |

In Table 7, the reaction system with the substrate concentration of 100 µM included 50 mM Tris-HCl (pH 7.5), 10 mM MgCl₂, 1 mM DTT, 100 µM g4, 100 µM g5, 200 µM ATP, and 0.2 mg/mL RNA ligase; a reaction temperature was 37 °C, and a reaction time was 16 h; and after the reaction completed, incubation was performed for 5 min at 80 °C, and then the reaction was terminated.

The reaction system with the substrate concentration of 300 µM included 50 mM Tris-HCl (pH 7.5), 10 mM MgCl₂, 1 mM DTT, 300 µM g4, 300 µM g5, 600 µM ATP, and 0.2 mg/mL RNA ligase; a reaction temperature was 37 °C, and a reaction time was 16 h; and after the reaction completed, incubation was performed for 5 min at 80 °C, and then the reaction was terminated.

### Embodiment 3

An RNA ligase ligated three fragments of single-stranded RNA to form gRNA of 100 nt under the guidance of template DNA, and a reaction schematic diagram was shown in C in Fig. 1.

A target synthetic sequence 1 in this embodiment: SEQ ID NO: 28:

GACUGAUAUACACAGGCCGAUGUGGUUUUAGAGCUAGAAAUAGCAAGUUAAAAUAAGG
CUAGUCCGUUAUCAACUUGAAAAAGUGGCACCGAGUCGGUGC.

Positions 2' of all ribonucleotides in the above sequence were methoxy modifications.

A ligating reaction was performed with the RNA ligase; g1, P-g2 (5' phosphorylated g2), and g3 fragments were ligated under the guidance of g-D (template nucleic acid strand); a reaction system included 50 mM Tris-HCl (pH 7.5), 10 mM MgCl₂, 1 mM DTT, 60 µM g1, 60 µM P-g2, , 60 µM g3, 60 µM g-D, 180 µM ATP, and 0.2 mg/mL RNA ligase; a reaction temperature was 16 °C, and a reaction time was 16 h; and after the reaction completed, incubation was performed for 5 min at 80 °C, and then the reaction was terminated; and DNase I with a final concentration of 0.1 U/µL was added, and digestion was performed for 4 h at 37 °C to remove a g-D splint.

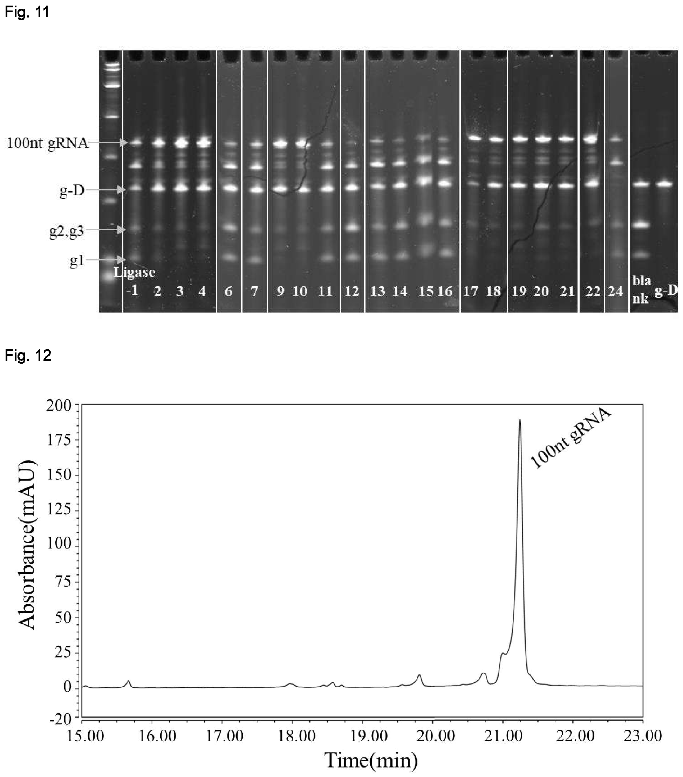

The reaction system was detected with urea-denatured polyacrylamide gel electrophoresis. Gel electrophoresis detection results were shown in Fig. 11, blank was a negative control group without adding the RNA ligase, and g-D was a control group only added with the template nucleic acid strand. Compared with the blank, new RNA bands were detected in the reaction system of the RNA ligases ligase-1, ligase-2, ligase-3, ligase-4, ligase-6, ligase-7, ligase-9, ligase-10, ligase-11, ligase-12, ligase-13, ligase-14, ligase-15, ligase-16, ligase-17, ligase-18, ligase-19, ligase-20, ligase-21, ligase-22, and ligase-24 between 80 nt and 150 nt on a RNA Marker. The molecular weight of the new band detected through liquid chromatography-mass spectrometry analysis was consistent with the theoretical molecular weight of the gRNA product.

The reaction system was detected using liquid chromatography (UPLC). Results were shown in Fig. 12. The catalytic activity of the RNA ligase through UPLC detection was shown in the table below (Table 8).

**Table 8**

| RNA ligase name | Sequence No. | Purity of 100nt gRNA product system |
|---|---|---|
| ligase-1 | SEQ ID NO: 1 | ++ |
| ligase-2 | SEQ ID NO: 2 | +++ |
| ligase-3 | SEQ ID NO: 3 | ++++ |
| ligase-4 | SEQ ID NO: 4 | ++++ |
| ligase-6 | SEQ ID NO: 6 | + |
| ligase-7 | SEQ ID NO: 7 | ++ |
| ligase-9 | SEQ ID NO: 9 | +++++ |
| ligase-10 | SEQ ID NO: 10 | +++++ |
| ligase-11 | SEQ ID NO: 11 | ++ |
| ligase-12 | SEQ ID NO: 12 | + |
| ligase-13 | SEQ ID NO: 13 | ++ |
| ligase-14 | SEQ ID NO: 14 | + |
| ligase-15 | SEQ ID NO: 15 | + |
| ligase-16 | SEQ ID NO: 16 | + |
| ligase-17 | SEQ ID NO: 17 | ++++ |
| ligase-18 | SEQ ID NO: 18 | +++++ |
| ligase-19 | SEQ ID NO: 19 | +++++ |
| ligase-20 | SEQ ID NO: 20 | +++++ |
| ligase-21 | SEQ ID NO: 21 | ++++ |
| ligase-22 | SEQ ID NO: 22 | ++++ |
| ligase-24 | SEQ ID NO: 24 | + |

| | | |
|---|---|---|
| Note: "+" indicated 0-20% (excluding an endpoint value of 0%) purity of the product system; "++" indicated 20-40% (excluding an endpoint value of 20%) purity of the product system; "+++" indicated 40-60% (excluding an endpoint value of 40%) purity of the product system; "++++" indicated 60-80% (excluding an endpoint value of 60%) purity of the product system; and "+++++" indicated 80-100% (excluding an endpoint value of 80%) purity of the product system. In the present application, "the purity of the product system" indicated that in the reaction system, a ratio of a target product to a total component (target product + remaining substrate) in the reaction system might be calculated by the area under the curve of different components in liquid chromatography results. | | |

Sequences of g-D and P-g2 were shown in Table 9.

**Table 9**

| Serial number | Sequence (5'->3') | Base number | 5' end modification | 3' end modification |
|---|---|---|---|---|
| g-D (SEQ ID NO: 37) | dTdGdAdTdAdAdCdGdGdAdCdTdAdG dCdCdTdTdAdTdTdTdTdAdAdCdTdT dGdCdTdAdTdTdTdCdTdAdGdCdTdC dTdAdAdAdAdCdCdAdCdAdTdCdGdG dCdCdTdG | 60nt | Phosphate group | Hydroxyl group |
| P-g2 (SEQ ID NO: 38) | GmUmUmUmUmAmGmAmGmCmUm AmGmAmAmAmUmAmGmCmAmAm GmUmUmAmAmAmAmUmAmAmGm GmCmUm | 36nt | Phosphate group | Hydroxyl group |

The m after A, C, G, or U represented the 2' methoxy modification for the ribonucleotide; the d before A, C, G, or T represented that the nucleotide was deoxyribonucleotide (DNA); g-D contained 12 nucleotides that was designed according to the CRISPR target fragment; based on the principle of complementary base pairing, the corresponding template nucleic acid strand included a variable sequence with 3-25 nt; the template nucleic acid strand included from the 5' end to the 3' end: a) a sequence that could be complementary paired with the 5' end of the substrate g3; b) a sequence that could be complementary paired with the substrate P-g2; and c) a sequence that could be complementary paired with the 3' end of the substrate g1. The template nucleic acid strand included the variable sequence with 3-25 nt. That is, the number of N on the 3' end of the template nucleic acid strand might be optionally selected as any integer among 3-25, including 3, 4, 5, 6, 7, 8, 9, 10, 11, 12, 13, 14, 15, 16, 17, 18, 19, 20, 21, 22, 23, 24, or 25, including but not limited to sequence shown in SEQ ID NO: 39 or SEQ ID NO: 40.

TGATAACGGACTAGCCTTATTTTAACTTGCTATTTCTAGCTCTAAAACNNN(SEQ ID NO: 39),

TGATAACGGACTAGCCTTATTTTAACTTGCTATTTCTAGCTCTAAAACNNNNNNNNNNNNNN
NNNNNNNNNNNN(SEQ ID NO: 40).

### Embodiment 4

An ssRNA ligase ligated three fragments of single-stranded RNA to form gRNA of 142 nt, and a reaction schematic diagram was shown in A in Fig. 1.

A target synthetic sequence 2 in this embodiment: SEQ ID NO: 41:

UGAUAUACACAGGCCGAUGUGGUUGUAGCUCCCUUUCUCAUUUCGGAAACGAAAUGA
GAACCGUUGCUACAAUAAGGCCGUCUGAAAAGAUGUGCCGCAACGCUCUGCCCCUUAAAG
CCUCUGCUUUAAGGGGCAUCGUUUC.

Positions 2' of all ribonucleotides in the above sequence shown in SEQ ID NO: 41 were methoxy modifications.

The 5' end of the above sequence shown in SEQ ID NO: 41 included a 21 nt variable sequence that was designed according to the CRISPR target fragment. The length of the variable sequence was 15-25 nt. That is, the number of N on the 5' end of the gRNA might be optionally selected as any integer among 15-25, including 15, 16, 17, 18, 19, 20, 21, 22, 23, 24, or 25, including but not limited to sequence shown in SEQ ID NO: 42 or SEQ ID NO: 43.
SEQ ID NO: 42: 
NNNNNNNNNNNNNNNGUUGUAGCUCCCUUUCUCAUUUCGGAAACGAAAUGAGAACCGU
UGCUACAAUAAGGCCGUCUGAAAAGAUGUGCCGCAACGCUCUGCCCCUUAAAGCCUCUGC
UUUAAGGGGCAUCGUUUC;
SEQ ID NO: 43:

NNNNNNNNNNNNNNNNNNNNNGUUGUAGCUCCCUUUCUCAUUUCGGAAACGAAA
UGAGAACCGUUGCUACAAUAAGGCCGUCUGAAAAGAUGUGCCGCAACGCUCUGCCCCUUA
AAGCCUCUGCUUUAAGGGGCAUCGUUUC.

Step 1(S1): substrates fragments Ng2 and Ng3 were ligated with an RNA ligase.

A ligating reaction was performed with the RNA ligase; a reaction system included 50 mM Tris-HCl (pH 7.5), 10 mM MgCl₂, 1 mM DTT, 50 µM Ng2, 50 µM Ng3, 200 µM ATP, and 0.2 mg/mL RNA ligase; a reaction temperature was 37 °C, and a reaction time was 16 h; and after the reaction completed, incubation was performed for 5 min at 80 °C, and then the reaction was terminated to obtain an intermediate product Ng2Ng3.

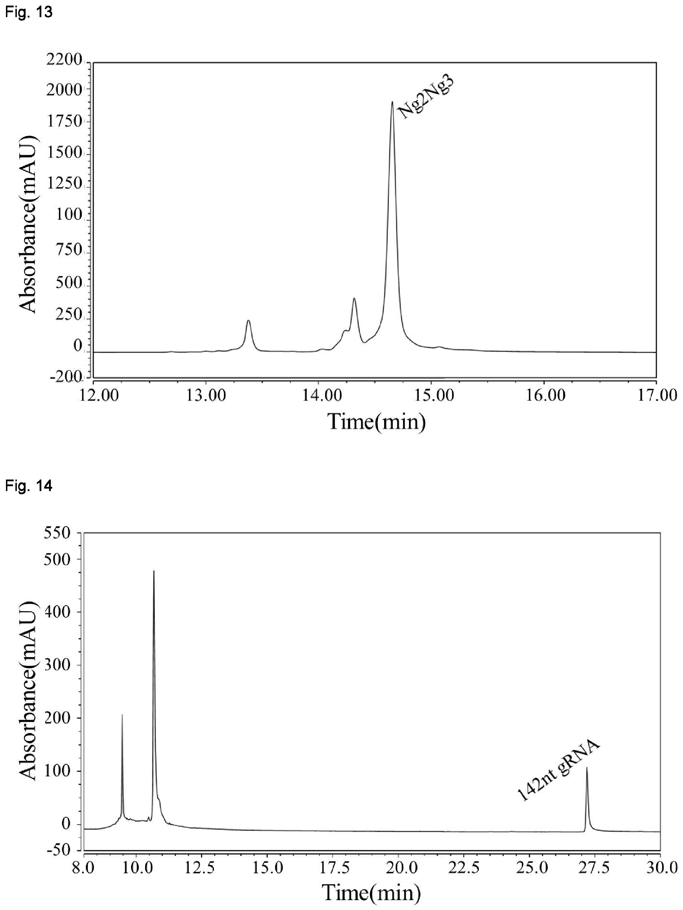

Through liquid chromatography (UPLC) analysis, results were shown in Fig. 13. The generation of new peaks was detected in the reaction system of the RNA ligases ligase-1, ligase-2, ligase-3, ligase-4, ligase-5, ligase-6, ligase-7, ligase-8, ligase-9, ligase-10, ligase-11, ligase-12, ligase-13, ligase-14, ligase-15, ligase-16, ligase-17, ligase-18, ligase-19, ligase-20, and ligase-21. Through mass spectrometry identification, the molecular weight of the new peak was consistent with the theoretical molecular weight of the ligation product of Ng2 and Ng3.

Step 2(S2): 5' phosphorylation was performed on the 84nt intermediate product Ng2Ng3.

5' end phosphorylation was performed on Ng2Ng3 with the method of S2 in Embodiment 1. P-Ng2Ng3 (Ng2Ng3 with 5' end phosphorylated) was prepared after purification. Through mass spectrometry identification, the molecular weight of the phosphorylated product was correct.

Step 3(S3): Ng1 and P-Ng2Ng3 fragments were ligated with the RNA ligase.

The reaction system including 50 mM Tris-HCl (pH 7.5), 10 mM MgCl₂, 1 mM DTT, 20 µM Ng1, 20 µM P-Ng2Ng3, 40 µM ATP, and 0.2 mg/mL RNA ligase was prepared; a reaction temperature was 37 °C, and a reaction time was 16 h; and after the reaction completed, incubation was performed for 5 min at 80 °C, and then the reaction was terminated to obtain a product gRNA.

Through liquid chromatography (UPLC) analysis, results were shown in Fig. 14. The generation of new peaks was detected in the reaction system of the RNA ligases ligase-1, ligase-2, ligase-3, ligase-4, ligase-5, ligase-6, ligase-7, ligase-8, ligase-9, ligase-10, ligase-11, ligase-12, ligase-13, ligase-14, ligase-15, ligase-16, ligase-17, ligase-18, ligase-19, ligase-20, and ligase-21. Through mass spectrometry identification, the molecular weight of the new peak was consistent with the theoretical molecular weight of the gRNA (142 nt) produced by the ligation of Ng1 and P-Ng2Ng3.

Sequences of Ng1, Ng2, and Ng3 were shown in Table 10.

**Table 10**

| Serial number | Sequence (5'->3') | Base number | 5' end modification | 3' end modification |
|---|---|---|---|---|
| Ng1 (SEQ ID NO: 44) | UmGmAmUmAmUmAmCmAmCmAmGmGm CmCmGmAmUmGmUmGmGmUmUmGmUm AmGmCmUmCmCmCmUmUmUmCmUmCm | 39nt | Hydroxyl group | Hydroxyl group |
| Ng2 | AmUmUmUmCmGmGmAmAmAmCmGmAm | 45nt | Hydroxyl | Hydroxyl |
| (SEQ ID NO: 45) | AmAmUmGmAmGmAmAmCmCmGmUmUm GmCmUmAmCmAmAmUmAmAmGmGmCm CmGmUmCmUmGm | | group | group |
| Ng3 (SEQ ID NO: 46) | AmAmAmAmGmAmUmGmUmGmCmCmGm CmAmAmCmGmCmUmCmUmGmCmCmCm CmUmUmAmAmAmGmCmCmUmCmUmGm CmUmUmUmAmAmGmGmGmGmCmAmUm CmGmUmUmUmCm | 58nt | Phosphate group | Modification |

The m after A, C, G, or U represented the 2' methoxy modification for the ribonucleotide; the 5' end of Ng1 contained 21 nucleotides (N) that might be designed according to the CRISPR target fragment; and corresponding substrate sequences included variable sequences consisting of 21 (optionally 15-25) nucleotides. That is, Ng1 included a sequence that was formed by ligating the variable sequence with 15-25 nt and a sequence shown in SEQ ID NO: 47, and the variable sequence was located in the 5' direction of the SEQ ID NO: 47.
SEQ ID NO: 47: GUUGUAGCUCCCUUUCUC.

### Embodiment 5

An RNA ligase ligated two fragments of 71nt single-stranded RNA to form gRNA of 142 nt, and a reaction schematic diagram was shown in B in Fig. 1.

A target synthetic sequence 2: SEQ ID NO: 41 in this embodiment:

UGAUAUACACAGGCCGAUGUGGUUGUAGCUCCCUUUCUCAUUUCGGAAACGAAAUGA
GAACCGUUGCUACAAUAAGGCCGUCUGAAAAGAUGUGCCGCAACGCUCUGCCCCUUAAAG
CCUCUGCUUUAAGGGGCAUCGUUUC.

Positions 2' of all ribonucleotides in the above sequence shown in SEQ ID NO: 41 were methoxy modifications.

A Ng4 and Ng5 ligating reaction was performed with the RNA ligase below; a reaction system included 50 mM Tris-HCl (pH 7.5), 10 mM MgCl₂, 1 mM DTT, 50 µM Ng4, 50 µM Ng5, 200 µM ATP, and 0.2 mg/mL RNA ligase; a reaction temperature was 37 °C, and a reaction time was 16 h; and after the reaction completed, incubation was performed for 5 min at 80 °C, and then the reaction was terminated to obtain a gRNA product.

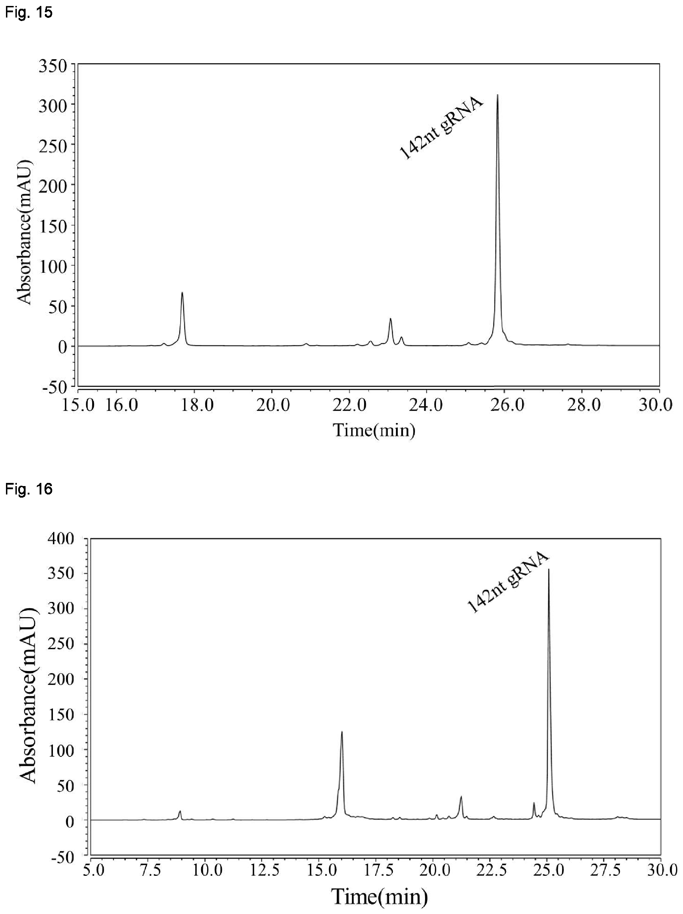

Through liquid chromatography (UPLC) analysis, results were shown in Fig. 15. The generation of new peaks was detected in the reaction system of the RNA ligases ligase-1, ligase-2, ligase-3, ligase-4, ligase-7, ligase-8, ligase-9, ligase-10, ligase-11, ligase-13, ligase-14, ligase-15, ligase-16, ligase-17, ligase-18, ligase-19, ligase-20, ligase-21, ligase-22, ligase-23, and ligase-24. Through mass spectrometry identification, the molecular weight of the new peak was consistent with the theoretical molecular weight of the gRNA (142 nt) produced by the ligation of Ng4 and Ng5.

Sequences of Ng4, and Ng5 were shown in Table 11.

**Table 11**

| Serial number | Sequence (5'->3') | Base number | 5' end modification | 3' end modification |
|---|---|---|---|---|
| Ng4 (SEQ ID NO: 48) | UmGmAmUmAmUmAmCmAmCmAmGmGm CmCmGmAmUmGmUmGmGmUmUmGmUm AmGmCmUmCmCmCmUmUmUmCmUmCm AmUmUmUmCmGmGmAmAmAmCmGmAm AmAmUmGmAmGmAmAmCmCmGmUmUm GmCmUmAmCmAm | 71nt | Hydroxyl group | Hydroxyl group |
| Ng5 (SEQ ID NO: 49) | AmUmAmAmGmGmCmCmGmUmCmUmGm AmAmAmAmGmAmUmGmUmGmCmCmGm CmAmAmCmGmCmUmCmUmGmCmCmCm CmUmUmAmAmAmGmCmCmUmCmUmGm CmUmUmUmAmAmGmGmGmGmCmAmUm CmGmUmUmUmCm | 71nt | Phosphate group | Modification |

The m after A, C, G, or U represented the 2' methoxy modification for the ribonucleotide; Ng4 contained 21 nucleotides that might be designed according to the CRISPR target fragment; and corresponding substrate sequences included variable sequences consisting of 21 (optionally 15-25) nucleotides. That is, Ng4 included a sequence that was formed by ligating the variable sequence with 15-25 nt and a sequence shown in SEQ ID NO: 50, and the variable sequence was located in the 5' direction of the SEQ ID NO: 50.
SEQ ID NO: 50:
GUUGUAGCUCCCUUUCUCAUUUCGGAAACGAAAUGAGAACCGUUGCUACA.

### Embodiment 6

An RNA ligase ligated three fragments of single-stranded RNA to form gRNA of 142 nt under the guidance of template DNA, and a reaction schematic diagram was shown in C in Fig. 1.

A target synthetic sequence 2 in this embodiment: SEQ ID NO: 41:

UGAUAUACACAGGCCGAUGUGGUUGUAGCUCCCUUUCUCAUUUCGGAAACGAAAUGA
GAACCGUUGCUACAAUAAGGCCGUCUGAAAAGAUGUGCCGCAACGCUCUGCCCCUUAAAG
CCUCUGCUUUAAGGGGCAUCGUUUC.

Positions 2' of all ribonucleotides in the above sequence shown in SEQ ID NO: 41 were methoxy modifications.

A ligating reaction was performed with the RNA ligase; Ng1, P-Ng2 (5' phosphorylated g2), and Ng3 fragments were ligated under the guidance of Ng-D (template nucleic acid strand); a reaction system included 50 mM Tris-HCl (pH 7.5), 10 mM MgCl₂, 1 mM DTT, 60 µM Ng1, 60 µM P-Ng2, , 60 µM Ng3, 60 µM Ng-D, 180 µM ATP, and 0.2 mg/mL RNA ligase; a reaction temperature was 16 °C, and a reaction time was 16 h; and after the reaction completed, incubation was performed for 5 min at 80 °C, and then the reaction was terminated.

Through liquid chromatography (UPLC) analysis, results were shown in Fig. 16. The generation of new peaks was detected in the reaction system of the RNA ligases ligase-1, ligase-2, ligase-3, ligase-4, ligase-6, ligase-7, ligase-9, ligase-10, ligase-11, ligase-12, ligase-13, ligase-14, ligase-15, ligase-16, ligase-17, ligase-18, ligase-19, ligase-20, ligase-21, ligase-22, and ligase-24. Through mass spectrometry identification, the molecular weight of the new peak was consistent with the theoretical molecular weight of the gRNA (142 nt) produced by the ligation of Ng1, P-Ng2 and Ng3.

Sequences of Ng-D and P-Ng2 were shown in Table 12.

**Table 12**

| Serial number | Sequence (5'->3') | Base number | 5' end modification | 3' end modification |
|---|---|---|---|---|
| Ng-D (SEQ ID NO: 51) | dGdGdCdAdCdAdTdCdTdTdTdTdCdAdG dAdCdGdGdCdCdTdTdAdTdTdGdTdAdG dCdAdAdCdGdGdTdTdCdTdCdAdTdTdT dCdGdTdTdTdCdCdGdAdAdAdTdGdAdG dAdAdAdGdGdGdAdGdC | 69nt | Phosphate group | Hydroxyl group |
| P-Ng2 (SEQ ID NO: 52) | AmUmUmUmCmGmGmAmAmAmCmGm AmAmAmUmGmAmGmAmAmCmCmGm UmUmGmCmUmAmCmAmAmUmAmAm GmGmCmCmGmUmCmUmGm | 45nt | Phosphate group | Hydroxyl group |

The m after A, C, G, or U represented the 2' methoxy modification for the ribonucleotide; The d before A, C, G, or T represented that the nucleotide was deoxynucleotide (DNA); the template nucleic acid strand Ng-D included from the 5' end to the 3' end: a) a sequence that could be complementary paired with the 5' end of the substrate Ng3; b) a sequence that could be complementary paired with the substrate P-Ng2; and c) a sequence that could be complementary paired with the 3' end of the substrate Ng1.

It may be seen from the above description that, in the above embodiments of the present disclosure, the following technical effects are realized. With the above method, two or more than two substrates can be ligated by the RNA ligases of the families such as Rnl1, RnI2, etc. with or without the help of the template nucleic acid strand. The nick position for ligating may be located on the stem-loop structure of the gRNA, or may also be located to the ligating region between the stem-loop structures, such that the plurality of substrates are ligated into a complete RNA strand, and complementary base pairing can be automatically performed between the substrates, thereby obtaining the gRNA having a secondary structure through preparation. Compared to solid-phase synthesis methods in the prior art, the method of the present disclosure does not need a solid carrier, and is relatively-high in product purity, large in difference in chain length between the product and impurities, easy to purify, and free of branching impurities. Compared to in-vitro transcription methods in the prior art, the products prepared by the method of the present disclosure is relatively-high in purity and easy to purify, and have specific 5' and 3' ends.

The above are only the preferred embodiments of the present disclosure and are not intended to limit the present disclosure. For person skilled in the art, the present disclosure may have various modifications and variations. Any modifications, equivalent replacements, improvements and the like made within the spirit and principle of the present disclosure all fall within the scope of protection of the present disclosure.

## Claims

1. A method for biosynthesizing a gRNA, wherein the method comprises:
ligating 3' end and 5' end of different substrates to form a phosphodiester bond with an RNA ligase, so as to form the gRNA, wherein the gRNA comprises a natural gRNA or a non-natural gRNA, and the number of the substrates is ≥2;
wherein, a ligation site of the substrates is located at any one or more positions of a stem-loop structure complementary region, a stem-loop structure loop region, or a linker region between stem-loop structures of the gRNA,
and wherein complementary base pairing occurs inside or between the substrates to form a secondary structure of the gRNA; and
the RNA ligase comprises any one or more of RNA ligase family 1, 2, or 3.

2. The method of claim 1, wherein the RNA ligase comprises:
one or more of the RNA ligase as shown in SEQ ID NO: 1, SEQ ID NO: 2, SEQ ID NO: 3, SEQ ID NO: 4, SEQ ID NO: 5, SEQ ID NO: 6, SEQ ID NO: 7, SEQ ID NO: 8, SEQ ID NO: 9, SEQ ID NO: 10, SEQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO: 18, SEQ ID NO: 19, SEQ ID NO: 20, SEQ ID NO: 21, SEQ ID NO: 22, SEQ ID NO: 23 or SEQ ID NO: 24; or
an enzyme having more than 70% identity with the RNA ligase as shown in any one of SEQ ID NO: 1 - SEQ ID NO: 24.

3. The method of claim 1, wherein the number of the substrates is two or more.

4. The method of claim 1, wherein the gRNA has a length of 67-146 nt.

5. The method of claim 1, wherein the sequence of the gRNA comprises a nucleotide sequence as shown in any one of SEQ ID NO: 25, SEQ ID NO: 26, SEQ ID NO: 27, SEQ ID NO: 28, SEQ ID NO: 41, SEQ ID NO: 42 or SEQ ID NO: 43.

6. The method of claim 5, wherein the substrates comprise:
SEQ ID NO: 29 or a nucleotide sequence consisting of 15-25 N nucleotides, and nucleotide sequences as shown in SEQ ID NO: 30 and SEQ ID NO: 31; or
a nucleotide sequence as shown in SEQ ID NO: 32 or a sequence comprising the nucleotide sequence consisting of 15-25 N nucleotides and a nucleotide sequence as shown in SEQ ID NO: 36, and a nucleotide sequence as shown in SEQ **ID** NO: 33; wherein the nucleotide sequence consisting of 15-25 N nucleotides is in the 5' direction of the nucleotide sequence as shown in SEQ **ID** NO: 36; or
a nucleotide sequence as shown in SEQ **ID** NO: 44 or a sequence comprising the nucleotide sequence consisting of 15-25 N nucleotides and a nucleotide sequence as shown in SEQ ID NO: 47, and nucleotide sequences as shown in SEQ **ID** NO: 45 and SEQ **ID** NO: 46; wherein the nucleotide sequence consisting of 15-25 N nucleotides is in the 5' direction of the nucleotide sequence as shown in SEQ **ID** NO: 36; or
a nucleotide sequence as shown in SEQ **ID** NO: 48 or a sequence comprising the nucleotide sequence consisting of 15 to 25 N nucleotides and a nucleotide sequence as shown in SEQ ID NO: 50, and a nucleotide sequence as shown in SEQ **ID** NO: 49; wherein the nucleotide sequence consisting of 15-25 N nucleotides is in the 5' direction of the nucleotide sequence as shown in SEQ **ID** NO: 50.

7. The method of claim 3, wherein when the number of the substrates is 2, the method comprises:
mixing the substrates and the RNA ligase, wherein the substrates comprise a first substrate and a second substrate, the RNA ligase links a phosphate group at the 5' end of the first substrate and a hydroxyl group at the 3' end of the second substrate to form the phosphodiester bond, thereby obtaining the gRNA.

8. The method of claim 7, wherein the 5' end of the second substrate is a 5' end protecting group, and the 3' end of the first substrate is a 3' end protecting group.

9. The method of claim 3, wherein when the number of the substrates is 3, the method comprises:
mixing a first substrate, a second substrate and the RNA ligase, wherein the 5' end of the first substrate is a phosphate group, the 3' end of the first substrate is a 3' end protecting group, and the 5' end and the 3' end of the second substrate are a hydroxyl group,
ligating the phosphate group at the 5' end of the first substrate and the hydroxyl group at the 3' end of the second substrate to form the phosphodiester bond with the RNA ligase, so as to obtain an intermediate product of which the 5' end is a hydroxyl group;
performing 5' phosphorylation on the intermediate product to obtain a 5' phosphorylated intermediate product;
mixing the 5' phosphorylated intermediate product, a third substrate and the RNA ligase, and ligating the phosphate group at the 5' end of the 5' phosphorylated intermediate product and the hydroxyl group at the 3' end of the third substrate to form the phosphodiester bond with the RNA ligase, so as to obtain the gRNA;
wherein the 5' end of the third substrate is a 5' end protecting group.

10. The method of claim 9, wherein further comprising repeatedly performing the ligating and the 5' phosphorylation to sequentially link four or more substrates with the method, wherein during each ligating step, two substrates are ligated, and the substrates are sequentially ligated to obtain the gRNA.

11. The method of claim 1, comprising using a template nucleic acid strand to guide the ligating of the substrates;
wherein the template nucleic acid strand has at least 3 base specific bindings with each of different substrates, the bases of the substrates specifically binding to the template nucleic acid chain are adjacent and form a nick, and obtain a double-stranded nucleotide structure containing the nick, bases adjacent to two ends of the nick are a 5' end and a 3' end of different substrates, the 5' end is a 5' phosphate, and the 3' end is a hydroxyl group;
ligating the 5' phosphate and the hydroxyl group at upstream and downstream of the nick to form the phosphodiester bond with the RNA ligase, and obtaining a double-stranded nucleotide structure; and
digesting the template nucleic acid strand of the double-stranded nucleotide structure with a DNase to obtain the gRNA.

12. The method of claim 11, wherein a sequence of the template nucleic acid strand comprises any one of the nucleotide sequences as shown in SEQ ID NO: 37, SEQ ID NO: 39, SEQ ID NO: 40, or SEQ ID NO: 51.

13. The method of claim 11, wherein after mixing the template nucleic acid strand with different substrates, incubating at a constant temperature or incubating at a 95°C and then cooling and annealing, to obtain the double-stranded nucleotide structure containing the nick;
wherein a temperature of the constant temperature incubation is 4-37 °C, and a time of the constant temperature incubation is ≥10 min; and
incubating at 95°C and then cooling and annealing comprises: 2 min incubation at 95 °C, decreasing the temperature to 12 °C at a rate of 0.5-1.2 °C/min, 10 min incubation at 12 °C, and then decreasing the temperature to 4 °C.

14. The method of claim 11, wherein the 3' end of the substrate at the 3' end of the gRNA is a 3' end protecting group, and the 5' end of the substrate at the 5' end of the gRNA is a 5' end protecting group.

15. The method of claim 1, wherein the secondary structure of the gRNA is formed by spontaneous complementary base pairing within the substrates or between the substrates.

16. The method of claim 9 or 14, wherein the 3' end protecting group comprises an N-acetylgalactosamine group, a phosphate group, -O-NH₂, an azide group, a cyanoethyl group, an allyl group or a 2-nitrobenzyl group; and the 5' end protecting group comprises a hydroxyl group, a hydrogen or a methyl group.

17. The method of any one of claims 1 to 15, wherein the substrate comprises one or more non-natural nucleotides, and the non-natural nucleotide comprises: a ribonucleotide having one or more of a ribose 2' position modification, a ribose backbone modification, a base modification or a phosphate backbone modification;
the ribose 2' position modification comprises a 2'-methoxy modification, a 2'-fluoro modification, a 2'-hydrogen modification, a 2'-methoxyethyl modification, a 2'-FANA modification, a locked nucleic acid modification, or a hexitol nucleic acid modification;
the ribose backbone modification comprises replacing a ribose in the nucleotide with ribuloNA, TNA, tPhoNA, or dXNA;
the base modification comprises a deazaadenine C7 modification, a deazaguanosine C7 modification, a cytosine C5 modification or a uridine C5 modification; and
the phosphate backbone modification comprises a PS modification.

18. A gRNA obtained with the method of any one of claims 1 to 17.
